# EUROPEAN PATENT APPLICATION

(11) **EP 4 282 327 A1**
(43) Date of publication of application: **29.11.2023**
(21) Application number: 22854936.6
(22) Date of filing: 20.04.2022
(51) Int. Cl.: A61B 5/0245

(54) **DEEP LEARNING-BASED HEART RATE MEASUREMENT METHOD AND WEARABLE DEVICE**

(30) Priority: 11.08.2021 CN 202110921225
(71) Applicant: Beijing Honor Device Co., Ltd., Beijing 100095 (CN)
(72) Inventor: ZHANG, Xiaowu, Shenzhen, Guangdong 518040 (CN); LI, Danhong, Shenzhen, Guangdong 518040 (CN); DI, Haoxuan, Shenzhen, Guangdong 518040 (CN)
(74) Representative: Beder, Jens
(86) International application number: PCT/CN2022/087841
(87) International publication number: WO 2023/015932

(57) **Abstract**

This application provides a deep learning-based heart rate detection method and a wearable device, and relates to the field of communications technologies. According to this solution, a deep Attention network is trained by using PPG and ACC sensor signals as inputs and using heart rate information and exercise scenario information as model outputs, and heart rate detection is performed by using a model obtained through training. Because motion artifact noise is distributed differently in different scenarios, a nonlinear relationship between a scenario, noise, and a heart rate may be fitted by using a deep Attention network learning mechanism, so that data denoising processing and feature extraction can be adaptively implemented in different scenarios, to achieve effects of signal denoising, signal fusion, and complex scenario identification in a plurality of complex scenarios, thereby canceling interference of motion artifact noise to a PPG signal, and improving precision of heart rate detection. Therefore, this solution can resolve a problem that a heart rate detection result is not precise because the PPG signal is interfered with by motion artifacts.

## Description

This application claims priority to Chinese Patent Application No. 202110921225.0, filed with the China National Intellectual Property Administration on August 11, 2021 and entitled "DEEP LEARNING-BASED HEART RATE DETECTION METHOD AND WEARABLE DEVICE", which is incorporated herein by reference in its entirety.

### TECHNICAL FIELD

This application relates to the field of communications technologies, and in particular, to a deep learning-based heart rate detection method and a wearable device.

### BACKGROUND

Heart rates are important indicators for showing a health status. A conventional measurement method is heart rate analysis based on an electrocardiograph (electrocardiograph, ECG). This method requires professional devices and knowledge, and cannot meet a daily measurement requirement of users. In recent years, with development and maturity of wearable devices (for example, smart wristband devices), a heart rate detection solution based on the wearable device becomes a mainstream.

The wearable device can calculate a heart rate by using a photoplethysmography (photoplethysmography, PPG) signal. A PPG sensor includes a light emitting diode (light emitting diode, LED) lamp and a receiver. The LED lamp constantly projects light onto skin, the light penetrates skin tissue and is absorbed by a blood flow, and the receiver receives a reflected optical signal. Because intensity of reflected light is related to a speed of the blood flow, and the speed of the blood flow is affected by a periodic heart rate, the PPG signal can reflect heart rate information.

However, because the PPG signal is susceptible to motion artifact interference, a result of heart rate detection based on the PPG signal is not accurate enough in an exercise state.

### SUMMARY

This application provides a deep learning-based heart rate detection method and a wearable device, to resolve a problem that a heart rate value detected in an exercise scenario by using a conventional heart rate algorithm based on a PPG signal is not precise enough.

To achieve the foregoing objective, the following technical solutions are used in this application.

According to a first aspect, this application provides a deep learning-based heart rate detection method, and the method includes:
when a user wears a wearable device, obtaining a photoplethysmography PPG signal and an acceleration ACC signal in response to a heart rate detection command;
obtaining heart rate information and scenario information based on ACC effective spectrum data corresponding to the ACC signal, PPG effective spectrum data corresponding to the PPG signal, and a target prediction model; and
displaying the heart rate information and the scenario information on a screen of the wearable device.

The target prediction model is a model that is obtained by training a deep attention Attention network by using ACC sample data and PPG sample data as inputs and using a heart rate label and an exercise scenario label as target variables, and the target prediction model has a scenario identification function and a heart rate prediction function.

According to this solution, the deep Attention network is trained by using the PPG and ACC sensor signals as inputs and using the heart rate information and the exercise scenario information as model outputs, and heart rate detection is performed by using a model obtained through training. Because motion artifact noise is distributed differently in different scenarios, a nonlinear relationship between a scenario, noise, and a heart rate may be fitted by using a deep Attention network learning mechanism, so that data denoising processing and feature extraction can be adaptively implemented in different scenarios, to achieve effects of signal denoising, signal fusion, and complex scenario identification in a plurality of complex scenarios, thereby canceling interference of motion artifact noise to a PPG signal, and improving precision of heart rate detection. Therefore, this solution can resolve a problem that a heart rate detection result is not precise because the PPG signal is interfered with by motion artifacts.

In some possible implementations of the first aspect, the method further includes:
when the heart rate information and the scenario information are obtained, recording the heart rate information and the scenario information in a label buffer; and
recording, in a data buffer, spectrum peak-point data corresponding to the heart rate information and the scenario information.

The spectrum peak-point data corresponding to the heart rate information and the scenario information includes a peak point location and an amplitude that are of a PPG spectrum and a peak point location and an amplitude that are of an ACC spectrum.

According to this solution, a model may be first trained in an initial period of heart rate detection, heart rate information and scenario information obtained by the model are stored, and corresponding peak locations and amplitudes of PPG frequency domain data and ACC frequency domain data are stored. Then, in a later period of heart rate detection, the stored data can be directly invoked when a condition is met. This can save calculation resources, and reduce a delay, so that a heart rate value can be obtained more quickly.

In some possible implementations of the first aspect, the obtaining heart rate information and scenario information based on ACC effective spectrum data corresponding to the ACC signal, PPG effective spectrum data corresponding to the PPG signal, and a target prediction model includes:
obtaining first N pieces of peak-point spectrum data based on the PPG effective spectrum data and the ACC effective spectrum data; and
if no peak-point spectrum data in the first N pieces of peak-point spectrum data is prestored in the data buffer, obtaining the heart rate information and the scenario information based on the ACC effective spectrum data corresponding to the ACC signal, the PPG effective spectrum data corresponding to the PPG signal, and the target prediction model; or
if any peak-point spectrum data in the first N pieces of peak-point spectrum data is prestored in the data buffer, reading, from the label buffer, heart rate information and scenario information that correspond to the any peak-point spectrum data.

According to this solution, locations and amplitudes of first three highest peaks of the PPG frequency domain data and the ACC frequency domain data may be separately obtained, and whether current data has appeared in the data buffer is checked. If the current data has not appeared, the current data enters the model; or if the current data has appeared, corresponding heart rate information and scenario information stored in the label buffer are read. This can save calculation resources, and reduce a delay, so that a heart rate value can be obtained more quickly.

In some possible implementations of the first aspect, the obtaining heart rate information and scenario information based on ACC effective spectrum data corresponding to the ACC signal, PPG effective spectrum data corresponding to the PPG signal, and a target prediction model includes:
inputting the ACC effective spectrum data and the PPG effective spectrum data to a first prediction model to obtain the heart rate information and the scenario information.

The target prediction model is the first prediction model, the first prediction model is a model that is obtained by training the deep attention Attention network by using the ACC sample data and the PPG sample data as inputs and using the heart rate label and the exercise scenario label as target variables, and the first prediction model has the scenario identification function and the heart rate prediction function.

The foregoing solution provides a heart rate detection solution: The PPG frequency domain data and the ACC frequency domain data are used as inputs, and the exercise scenario information and the heart rate information are output. In this way, in actual implementation, the wearable device may output both current scenario information and a current heart rate value when detecting a heart rate. According to the solution in this application, a user behavior or an exercise state is considered, to support detection of heart rate values of the user in different behavior states (for example, a resting state and various exercise states). The solution in this application can resolve a problem of various types of noise interference caused to a PPG signal in different scenarios, to effectively detect a heart rate in different noise scenarios.

In some possible implementations of the first aspect, the obtaining heart rate information and scenario information based on ACC effective spectrum data corresponding to the ACC signal, PPG effective spectrum data corresponding to the PPG signal, and a target prediction model includes:
inputting the ACC effective spectrum data to a second prediction model to obtain the scenario information, where the second prediction model is a model that is obtained by training a deep neural network by using the ACC sample data as an input and using the exercise scenario label as a target variable, and the second prediction model has the scenario identification function; and
inputting the PPG effective spectrum data and the obtained scenario information to a third prediction model to obtain the heart rate information, where the third prediction model is a model that is obtained by training the deep Attention network by using the PPG sample data, the ACC sample data, and the exercise scenario label as inputs and using the heart rate label as a target variable, and the third prediction model has the heart rate detection function.

The target prediction model includes the second prediction model and the third prediction model.

The foregoing solution provides another heart rate detection solution: An exercise scenario information identification function and a heart rate information identification function are separated. Scenario information identification is first performed by using a deep neural network model, and then an identification result and frequency domain sensor data are used as deep Attention inputs to output a heart rate value. The solution in this application can resolve a problem of various types of noise interference caused to a PPG signal in different scenarios, to effectively detect a heart rate in different noise scenarios.

In some possible implementations of the first aspect, after the obtaining a PPG signal and an ACC signal, the method further includes:
separately performing first preprocessing on the ACC signal and the PPG signal to obtain the ACC effective spectrum data and the PPG effective spectrum data.

The first preprocessing includes fast Fourier transform FFT and filtering processing.

According to the foregoing solution, FFT transform and filtering processing may be performed on a collected signal to obtain an effective spectrum, and a heart rate value is detected by using the effective spectrum. Because an interference signal is removed from the effective spectrum, precision of heart rate detection can be improved.

In some possible implementations of the first aspect, the obtaining a PPG signal and an ACC signal includes:
collecting the PPG signal by using a PPG sensor, and collecting the ACC signal by using an acceleration sensor.

The PPG signal may be used to detect a heart rate, and the ACC signal may be used to identify an exercise scenario. According to the solution in this application, heart rate detection is performed by using the PPG signal and the ACC signal in combination, to resolve a problem of various types of noise interference caused to a PPG signal in different scenarios, thereby effectively detecting a heart rate in different noise scenarios.

In some possible implementations of the first aspect, the method further includes:
sending the heart rate information and the scenario information to a terminal device, where the terminal device is an electronic device wirelessly connected to the wearable device; and
displaying the heart rate information and the scenario information on a screen of the terminal device.

According to this solution, the heart rate information and the scenario information not only can be displayed by using the wearable device, but also can be displayed by using the terminal device connected to the wearable device, to help the user view a heart rate value, thereby improving use experience of a user.

According to a second aspect, this application provides a method for training a model used for heart rate detection, including:
obtaining a multi-scenario sample set, where the multi-scenario sample set is a data sample set obtained through detection based on a plurality of exercise scenarios;
extracting ACC sample data, PPG sample data, and a heart rate label from the multi-scenario sample set;
training a deep attention Attention network by using the ACC sample data and the PPG sample data as inputs and using the heart rate label and an exercise scenario label as target variables; and
obtaining a first prediction model, where the first prediction model has a scenario identification function and a heart rate prediction function.

According to the foregoing model training method provided in this application, a model obtained through training by using PPG frequency domain data and ACC frequency domain data as inputs and using exercise scenario information and heart rate information as targets has the scenario identification function and the heart rate prediction function. Because motion artifact noise is distributed differently in different scenarios, a nonlinear relationship between a scenario, noise, and a heart rate may be fitted by using a deep Attention network learning mechanism, so that data denoising processing and feature extraction can be adaptively implemented in different scenarios, to achieve effects of signal denoising, signal fusion, and complex scenario identification in a plurality of complex scenarios, thereby canceling interference of motion artifact noise to a PPG signal, and improving precision of heart rate detection. Therefore, this solution can resolve a problem that a heart rate detection result is not precise because the PPG signal is interfered with by motion artifacts.

In some possible implementations of the second aspect, the training a deep Attention network by using the ACC sample data and the PPG sample data as inputs and using the heart rate label and an exercise scenario label as target variables includes:
adjusting a parameter of a model through cross-validation, so that the model learns to predict heart rate information in different exercise scenarios.

According to the solution in this application, when a deep Attention network model is trained, a parameter of the model is constantly adjusted through cross-validation, until a training target is achieved. In this way, it can be ensured that the model obtained through training has a good heart rate prediction function, thereby improving precision of heart rate detection.

In some possible implementations of the second aspect, the obtaining a multi-scenario sample set includes:
connecting a wearable device and a heart rate band device to a data collection module;
when a user wearing the wearable device and the heart rate band device does a first exercise, obtaining, by the data collection module, first heart rate detection data of the wearable device and second heart rate detection data of the heart rate band device; and
when the user does a second exercise, obtaining, by the data collection module, third heart rate detection data of the wearable device and fourth heart rate detection data of the heart rate band device.

The first exercise and the second exercise are exercises indicated by the exercise scenario label, and the multi-scenario sample set includes the first heart rate detection data, the second heart rate detection data, the third heart rate detection data, and the fourth heart rate detection data.

For example, a plurality of exercise scenarios that need to be collected may be formulated, for example, walking, running, swimming, climbing, rope jumping, playing football, and playing basketball. It should be noted that the exercise scenario herein may cover a resting scenario.

When a subject exercises in a specific scenario, the heart rate band device collects heart rate label data in the specific scenario, where the heart rate label data may be used as a target value of model training; and the wearable device (for example, a wristband device) collects a PPG signal and an ACC signal in the specific scenario as to-be-trained model parameters.

In some possible implementations of the second aspect, the extracting ACC sample data and PPG sample data from the multi-scenario sample set includes:
extracting the ACC sample data and the PPG sample data from detection data of the wearable device.

The detection data of the wearable device includes the first heart rate detection data and the third heart rate detection data.

In some possible implementations of the second aspect, the extracting a heart rate label from the multi-scenario sample set includes:
extracting the heart rate label from detection data of the heart rate band device.

The detection data of the heart rate band device includes the second heart rate detection data and the fourth heart rate detection data.

According to the solution in this application, during module training, the heart rate label detected by the heart rate band device may be used as a target variable, so that a trained model has the heart rate prediction function.

In some possible implementations of the second aspect, after the extracting ACC sample data, PPG sample data, and a heart rate label from the multi-scenario sample set, the method further includes:
performing fast Fourier transform FFT and filtering processing on the ACC sample data and the PPG sample data to obtain filtered ACC sample data and PPG sample data.

The training a deep Attention network by using the ACC sample data and the PPG sample data as inputs and using the heart rate label and an exercise scenario label as target variables includes:
training the deep Attention network by using the filtered ACC sample data and PPG sample data as inputs and using the heart rate label and the exercise scenario label as target variables.

In some possible implementations of the second aspect, the filtering processing is used to filter out noise data outside [0.7 Hz, 4 Hz] in a spectrum.

In this way, effective spectrum data from which noise data is filtered out is used for model training, which helps cancel interference of motion artifact noise to a PPG signal.

In some possible implementations of the second aspect, after the obtaining a first prediction model, the method further includes:
quantizing the first prediction model by using a preset quantization parameter, to obtain a quantized first prediction model.

In this embodiment of this application, a trained model may be quantized by using a parameter of 8 Bits or 16 Bits, and network structure information and quantized parameter data of the trained model are stored. After the model is quantized, storage space of the wearable device is reduced, and a speed of calculation of an integer type is increased. Therefore, storage space of a component of the wearable device can be reduced, and a calculation speed can be increased.

According to a third aspect, this application provides a method for training a model used for heart rate detection, including:
obtaining a multi-scenario sample set, where the multi-scenario sample set is a data sample set obtained through detection based on a plurality of exercise scenarios;
extracting ACC sample data, PPG sample data, and a heart rate label from the multi-scenario sample set;
training a deep attention Attention network by using the ACC sample data, the PPG sample data, and an exercise scenario label as inputs and using the heart rate label as a target variable; and
obtaining a third prediction model, where the third prediction model has a heart rate prediction function.

According to the foregoing model training method provided in this application, a model obtained through training by using PPG frequency domain data, ACC frequency domain data, and scenario information as inputs and using heart rate information as a target has the heart rate prediction function. Because motion artifact noise is distributed differently in different scenarios, a nonlinear relationship between a scenario, noise, and a heart rate may be fitted by using a deep Attention network learning mechanism, so that data denoising processing and feature extraction can be adaptively implemented in different scenarios, to achieve effects of signal denoising, signal fusion, and complex scenario identification in a plurality of complex scenarios, thereby canceling interference of motion artifact noise to a PPG signal, and improving precision of heart rate detection. Therefore, this solution can resolve a problem that a heart rate detection result is not precise because the PPG signal is interfered with by motion artifacts.

In some possible implementations of the third aspect, the method further includes:
training a deep neural network by using the ACC sample data and the PPG sample data as inputs and using the exercise scenario label as a target variable, to obtain a second prediction model, where the second prediction model has a scenario identification function.

In some possible implementations of the third aspect, the training a deep Attention network by using the ACC sample data, the PPG sample data, and an exercise scenario label as inputs and using the heart rate label as a target variable includes:
adjusting a parameter of an Attention network model through cross-validation, so that the Attention network model learns to predict heart rate information in different exercise scenarios.

According to the solution in this application, when a deep Attention network model is trained, a parameter of the model is constantly adjusted through cross-validation, until a training target is achieved. In this way, it can be ensured that the model obtained through training has a good heart rate prediction function, thereby improving precision of heart rate detection.

In some possible implementations of the third aspect, the obtaining a multi-scenario sample set includes:
connecting a wearable device and a heart rate band device to a data collection module;
when a user wearing the wearable device and the heart rate band device does a first exercise, obtaining, by the data collection module, first heart rate detection data of the wearable device and second heart rate detection data of the heart rate band device; and
when the user does a second exercise, obtaining, by the data collection module, third heart rate detection data of the wearable device and fourth heart rate detection data of the heart rate band device.

The first exercise and the second exercise are exercises indicated by the exercise scenario label, and the multi-scenario sample set includes the first heart rate detection data, the second heart rate detection data, the third heart rate detection data, and the fourth heart rate detection data.

In some possible implementations of the third aspect, the extracting ACC sample data and PPG sample data from the multi-scenario sample set includes:
extracting the ACC sample data and the PPG sample data from detection data of the wearable device.

The detection data of the wearable device includes the first heart rate detection data and the third heart rate detection data.

In some possible implementations of the third aspect, the extracting a heart rate label from the multi-scenario sample set includes:
extracting the heart rate label from detection data of the heart rate band device.

The detection data of the heart rate band device includes the second heart rate detection data and the fourth heart rate detection data.

According to the solution in this application, during module training, the heart rate label detected by the heart rate band device may be used as a target variable, so that a trained model has the heart rate prediction function.

In some possible implementations of the third aspect, after the extracting ACC sample data, PPG sample data, and a heart rate label from the multi-scenario sample set, the method further includes:
performing fast Fourier transform FFT and filtering processing on the ACC sample data and the PPG sample data to obtain filtered ACC sample data and PPG sample data.

The training a deep Attention network by using the ACC sample data, the PPG sample data, and an exercise scenario label as inputs and using the heart rate label as a target variable includes:
training the deep Attention network by using the filtered ACC sample data and PPG sample data and the exercise scenario label as inputs and using the heart rate label as a target variable.

In some possible implementations of the third aspect, the filtering processing is used to filter out noise data outside [0.7 Hz, 4 Hz] in spectrum data corresponding to the ACC sample data and the PPG sample data.

In this way, effective spectrum data from which noise data is filtered out is used for model training, which helps cancel interference of motion artifact noise to a PPG signal.

In some possible implementations of the third aspect, after the obtaining a third prediction model, the method further includes:
quantizing the third prediction model by using a preset quantization parameter, to obtain a quantized third prediction model.

After the model is quantized, storage space of the wearable device is reduced, and a speed of calculation of an integer type is increased. Therefore, storage space of a component of the wearable device can be reduced, and a calculation speed can be increased.

According to a fourth aspect, this application provides a deep learning-based heart rate detection apparatus. The apparatus includes a unit configured to perform the method in the first aspect. The apparatus may correspondingly perform the method described in the first aspect. For related description of the unit in the apparatus, refer to the description in the first aspect. For brevity, details are not described herein again.

The method described in the first aspect, the second aspect, and/or the third aspect may be implemented by hardware, or may be implemented by executing corresponding software by hardware. The hardware or the software includes one or more modules or units corresponding to the foregoing function, for example, a processing module or unit or a display module or unit.

According to a fifth aspect, this application provides an electronic device. The electronic device includes a processor, and the processor is coupled to a memory. The memory is configured to store a computer program or instructions, and the processor is configured to execute the computer program or the instructions stored in the memory, so that the method in the first aspect is performed.

For example, the processor is configured to execute the computer program or the instructions stored in the memory, so that the apparatus performs the method in the first aspect.

According to a sixth aspect, this application provides a computer-readable storage medium. The computer-readable storage medium stores a computer program (also referred to as instructions or code) used to implement the method in the first aspect, the second aspect, and/or the third aspect.

For example, when the computer program is executed by a computer, the computer is enabled to perform the method in the first aspect.

According to a seventh aspect, this application provides a chip, including a processor. The processor is configured to read and execute a computer program stored in a memory, to perform the method in the first aspect, the second aspect, and/or the third aspect, and any possible implementation of the first aspect, the second aspect, and/or the third aspect.

Optionally, the chip further includes the memory, and the memory is connected to the processor by using a circuit or a wire.

According to an eighth aspect, this application provides a chip system, including a processor. The processor is configured to read and execute a computer program stored in a memory, to perform the method in the first aspect, the second aspect, and/or the third aspect, and any possible implementation of the first aspect, the second aspect, and/or the third aspect.

Optionally, the chip system further includes the memory, and the memory is connected to the processor by using a circuit or a wire.

According to a ninth aspect, this application provides a computer program product. The computer program product includes a computer program (also referred to as instructions or code). When the computer program is executed by a computer, the computer is enabled to implement the method in the first aspect, the second aspect, and/or the third aspect.

It may be understood that, for beneficial effects of the fourth aspect to the ninth aspect, refer to the related descriptions in the first aspect, the second aspect, and/or the third aspect. Details are not described herein again.

### BRIEF DESCRIPTION OF DRAWINGS

FIG. 1 is a schematic diagram of a system architecture applied to a deep learning-based heart rate detection method according to an embodiment of this application;
FIG. 2 is a schematic flowchart of a deep learning-based heart rate detection method according to an embodiment of this application;
FG. 3 is a schematic diagram of an offline model training procedure in a deep learning-based heart rate detection method according to an embodiment of this application;
FIG. 4 is a schematic diagram of a waveform of filter processing in a deep learning-based heart rate detection method according to an embodiment of this application;
FIG. 5 is a schematic diagram of an online model prediction procedure in a deep learning-based heart rate detection method according to an embodiment of this application;
FIG. 6 is a schematic flowchart of another deep learning-based heart rate detection method according to an embodiment of this application;
FIG. 7 is a schematic diagram of an offline model training procedure in another deep learning-based heart rate detection method according to an embodiment of this application;
FIG. 8 is a schematic diagram of an online model prediction procedure in another deep learning-based heart rate detection method according to an embodiment of this application;
FIG. 9A to FIG. 9C are schematic diagrams of interfaces applied to a deep learning-based heart rate detection method according to an embodiment of this application;
FIG. 10 is a schematic diagram of a structure of a deep learning-based heart rate detection apparatus according to an embodiment of this application; and
FIG. 11 is a schematic diagram of a structure of a wearable device according to an embodiment of this application.

### DESCRIPTION OF EMBODIMENTS

To make the objectives, technical solutions, and advantages of embodiments of this application clearer, the following clearly and completely describes the technical solutions in embodiments of this application with reference to the accompanying drawings in embodiments of this application. Clearly, the described embodiments are some rather than all of embodiments of this application. Based on embodiments of this application, all other embodiments obtained by a person of ordinary skill in the art without creative efforts fall within the protection scope of this application.

In this specification, the term "and/or" is an association relationship that describes associated obj ects, and indicates that three relationships may exist. For example, "A and/or B" may indicate the following three cases: only A exists, both A and B exist, and only B exists. The character "/" in this specification indicates an "or" relationship between associated objects. For example, "A/B" indicates A or B.

The terms "first", "second", and the like in the specification and the claims in this specification are used to distinguish between different objects, and are not used to describe a specific sequence of the objects. For example, first preprocessing and second preprocessing are used to distinguish between different preprocessing, and are not used to describe a specific sequence of the preprocessing.

In embodiments of this application, words such as "example" or "for example" are used to represent giving examples, illustrations, or descriptions. Any embodiment or design solution described as "example" or "for example" in embodiments of this application should not be construed as being more preferred or advantageous than other embodiments or design solutions. Exactly, use of the words such as "example" or "for example" is intended to present a related concept in a specific manner.

In the description of embodiments of this application, unless otherwise stated, "a plurality of" means two or more. For example, "a plurality of processing units" means two or more processing units, and "a plurality of elements" means two or more elements.

PPG is a non-intrusive detection technology that converts a human biological signal into an electrical signal by using an optical principle. Specifically, LED light is incident on skin, light reflected back or transmitted after a part of the LED light is absorbed by skin tissue is received by a photosensor, and an electrical signal obtained by the photosensor is converted into a digital signal to obtain a PPG signal.

PPG is non-invasive, simple, and portable, and also has other advantages, and therefore is widely used for health monitoring of a human body in terms of physiological heart rate, blood oxygen, and pressure. The heart rate is one of parameters used to measure a heart beating capability, and therefore accurate detection of the heart rate has important medical significance.

Ideally, similar to an ECG signal, the PPG signal can precisely detect a heart rate value of a human body at each moment. However, due to noise impact in complex scenarios such as hardware, temperature, and exercising, a real signal of PPG is distorted, and accuracy of heart rate calculation is greatly limited. A motion artifact has the largest impact. The motion artifact causes a loss or deformation of a peak and a valley on a PPG time domain signal, and consequently precision of a time-domain counting method is reduced. In addition, a PPG spectrum peak is abnormal in frequency domain, a dominant frequency of the PPG signal at a current moment cannot be precisely positioned, and it is difficult to obtain an accurate result. Therefore, how to adaptively cancel complex noise in the PPG signal and improve heart rate detection precision is an important problem.

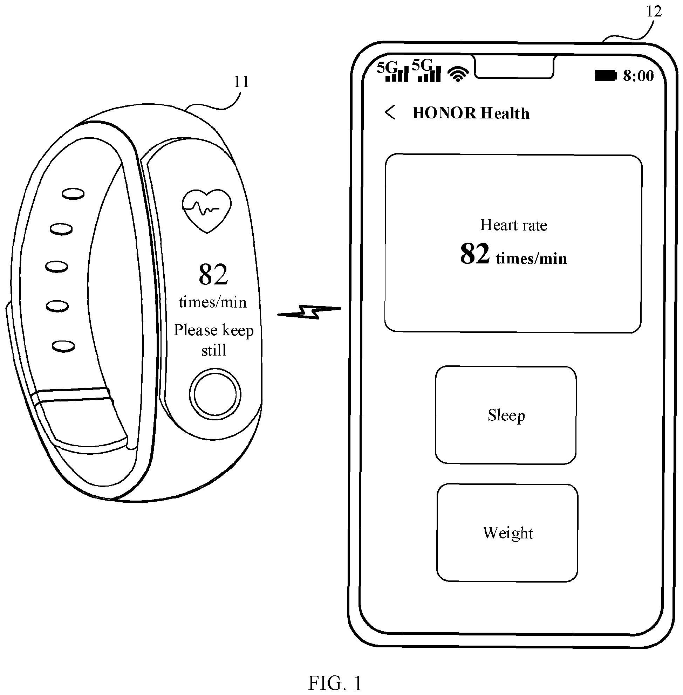

FIG. 1 is a schematic diagram of a system architecture related to example embodiments of this application. As shown in FIG. 1, the system architecture includes a wearable device 11. The wearable device may detect a human heart rate value by using a PPG technology. Optionally, the wearable device may display a heart rate value. Optionally, the system architecture may further include a terminal device 12 wirelessly connected to the wearable device 11. The terminal device 12 may receive and display a heart rate value sent by the intelligent wearable device 11, to provide the heart rate value for a user to view.

The wearable device 11 may be a user-wearable electronic device that supports heart rate detection, such as a smartwatch or a smart band. For ease of description, the following describes the wearable device 11 by using a smart band as an example.

However, a heart rate detection result obtained by a conventional heart rate algorithm in a resting state is relatively accurate, but heart rate detection in an exercise state is not accurate enough. As shown in FIG. 1, when performing heart rate detection, a smart band 11 displays prompt information: Please keep still. A heart rate value detected in an exercise state may not be precise due to exercise noise interference. A reason lies in the following: In the exercise state, a shift occurs between the smart band and skin, or the smart band jitters up and down or jitters left and right, and a watch compresses a blood vessel, or the like, which causes distortion of a heart rate detection signal. Therefore, a heart rate value detected by using the conventional heart rate algorithm is not precise enough.

Currently, a large amount of artificial noise preprocessing and rule formulation are performed for a signal in a conventional technology, a large amount of knowledge in the art is required as a support, and a good effect can be achieved only in a specific parameter and a specific scenario. As a result, an end-to-end learning mode cannot be implemented. A technical solution for adaptively cancelling noise and compensating for a signal under action of both various complex motion artifacts and noise cannot be found in the current technology.

In other words, a conventional signal processing method is seriously affected by the motion artifact, which causes low heart rate accuracy.

In view of this, according to a solution in the embodiments of this application, a deep Attention network is trained by using PPG and ACC sensor signals as inputs and using heart rate information and exercise scenario information as model outputs, and heart rate detection is performed by using a model obtained through training. Because motion artifact noise is distributed differently in different scenarios, a nonlinear relationship between a scenario, noise, and a heart rate may be fitted by using a deep Attention network learning mechanism, so that data denoising processing and feature extraction can be adaptively implemented in different scenarios, to achieve effects of signal denoising, signal fusion, and complex scenario identification in a plurality of complex scenarios, thereby canceling interference of motion artifact noise to a PPG signal, and improving precision of heart rate detection. Therefore, this solution can resolve a problem that a heart rate detection result is not precise because the PPG signal is interfered with by motion artifacts.

According to the solution in this application, a user behavior or an exercise state is considered, so that the solution in this application can support detection of heart rate values of a user in different exercise scenarios (for example, various exercise states, where a resting state is certainly also included). In addition, a current scenario and a heart rate value in the current scenario can be detected. According to the solution in this application, high-precision heart rate monitoring can be continuously performed in real time even if exercise noise interference exists.

### First Embodiment

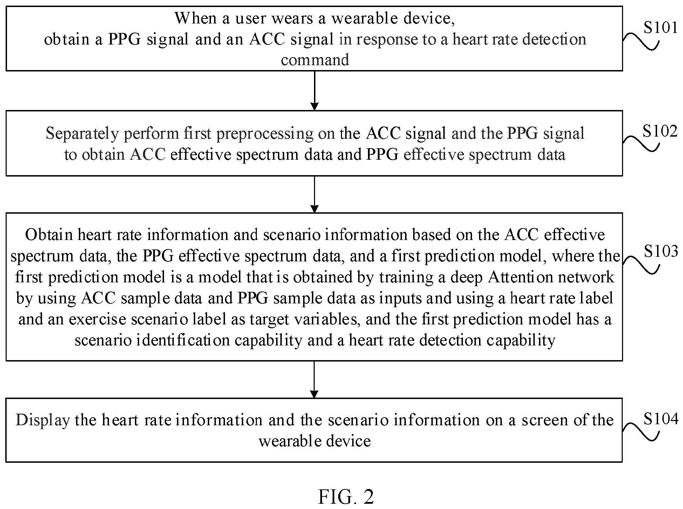

With reference to FIG. 2, the following describes in detail a deep learning-based heart rate detection method according to the first embodiment of this application.

FIG. 2 is a schematic flowchart of the heart rate detection method according to an embodiment of this application. As shown in FIG. 2, the method includes the following steps S101-S104.

S101: When a user wears a wearable device, obtain a PPG signal and an ACC signal in response to a heart rate detection command.

In this embodiment of this application, when the user wears the wearable device, if the wearable device is in an enabled state, the wearable device may be configured to detect a heart rate value of the user at each moment. A heart rate is a quantity of heart beats per minute of a normal person in a quiet state, which is also referred to as a quiet heart rate, and is usually 60~100 times/minute (bpm).

The wearable device may be provided with a PPG sensor and an acceleration sensor. The PPG sensor may obtain the PPG signal through detection, and the acceleration sensor may obtain the acceleration (acceleration, ACC) signal through detection. The ACC signal may reflect exercise data of the user, and the PPG signal may reflect heart rate data of the user.

For example, when a preset heart rate detection trigger condition is met, the wearable device is triggered to perform heart rate detection. For example, the wearable device receives the heart rate detection command, and in response to the heart rate detection command, triggers the PPG sensor to detect or collect the PPG signal, and triggers the acceleration sensor to detect or collect the ACC signal, so that the wearable device obtains the PPG signal and the ACC signal.

Optionally, in this embodiment of this application, the preset heart rate detection trigger condition may be any one of the following: The wearable device receives an operation of triggering, by the user, enabling of a heart rate detection function, the wearable device detects a heart rate in real time, and the wearable device periodically detects a heart rate and starts heart rate detection in an N^{th} period. This may be specifically determined based on an actual use requirement, and is not limited in this embodiment of this application.

S102: Separately perform first preprocessing on the ACC signal and the PPG signal to obtain ACC effective spectrum data and PPG effective spectrum data.

The first preprocessing may include Fourier transform (fast Fourier transform, FFT) and filtering processing. The filtering processing may be band-pass filtering processing.

In this embodiment of this application, after obtaining the ACC signal, the wearable device may perform FFT transform on the ACC signal to obtain a corresponding ACC spectrum, and then perform band-pass filtering processing on the ACC spectrum to filter out noise data to obtain an ACC effective spectrum.

Similarly, after obtaining the PPG signal, the wearable device may perform FFT transform on the PPG signal to obtain a corresponding PPG spectrum, and then perform band-pass filtering processing on the PPG spectrum to filter out noise data to obtain a PPG effective spectrum.

S103: Obtain heart rate information and scenario information based on the ACC effective spectrum data, the PPG effective spectrum data, and a first prediction model, where the first prediction model is a model that is obtained by training a deep attention (Attention) network by using ACC sample data and PPG sample data as inputs and using a heart rate label and an exercise scenario label as target variables, and the first prediction model has a scenario identification capability and a heart rate detection capability.

With a preset target task, the Attention network uses an attention mechanism to actively and consciously focus on a specific object, and selects only some key information inputs for processing, to improve efficiency of a neural network.

In this embodiment of this application, the wearable device may use the ACC effective spectrum data and the PPG effective spectrum data as input parameters of the first prediction model to perform an online prediction procedure by using the first prediction model. Through the first prediction model, the wearable device may identify whether a current exercise scenario of the user is resting or exercising; may precisely identify a specific type of exercise, for example, walking, swimming, running, or climbing; and may accurately detect a heart rate value of the user in the current exercise scenario.

For ease of description, herein, an exercise scenario identified by the wearable device is described as scenario information, and a heart rate value detected by the wearable device is described as heart rate information. In this way, after the online prediction procedure performed by using the first prediction model ends, the first prediction model may output both the scenario information and the heart rate information.

It should be noted that, the first prediction model provided in this embodiment of this application is a scenario identification and heart rate detection model that is obtained by training the deep Attention network based on the heart rate information and the exercise scenario information. To be specific, in the solution in this application, the exercise scenario information and the heart rate information are combined to perform model training, so that exercise noise can be reduced, to avoid impact of motion artifacts on heart rate detection accuracy. Therefore, a heart rate value output by the first prediction model is more precise.

An offline training procedure of the first prediction model is described in detail below, and details are not described herein.

S104: Display the heart rate information and the scenario information.

In a possible implementation, the wearable device may display the heart rate information and the scenario information.

In a possible implementation, when the wearable device maintains a wireless connection to a terminal device, if the wearable device obtains the heart rate information and the scenario information, the wearable device may send the heart rate information and the scenario information to the terminal device, and the terminal device displays the heart rate information and the scenario information for a user to view on the terminal device.

For example, when an HONOR Health APP is installed in the terminal device, the terminal device may display the heart rate information and the scenario information in an interface of the HONOR Health APP.

Certainly, in this embodiment of this application, only the heart rate information may be displayed, and the scenario information is not displayed. This may be specifically set based on an actual use requirement, and is not limited in this embodiment of this application.

It should be noted that, display styles, such as a font size and a font color, of the heart rate information and the scenario information are not limited in this embodiment of this application.

In the solution in this application, a deep Attention learning mechanism is used, so that a plurality of exercise scenarios can be identified, corresponding adaptive fitting between a scenario and noise can be performed, and excessive data preprocessing and manual experience parameter formulation are not required. The adaptive deep Attention network can be used to monitor a heart rate in real time, to resolve a problem of various types of noise interference caused to a PPG signal in different scenarios, thereby effectively detecting a heart rate in different noise scenarios.

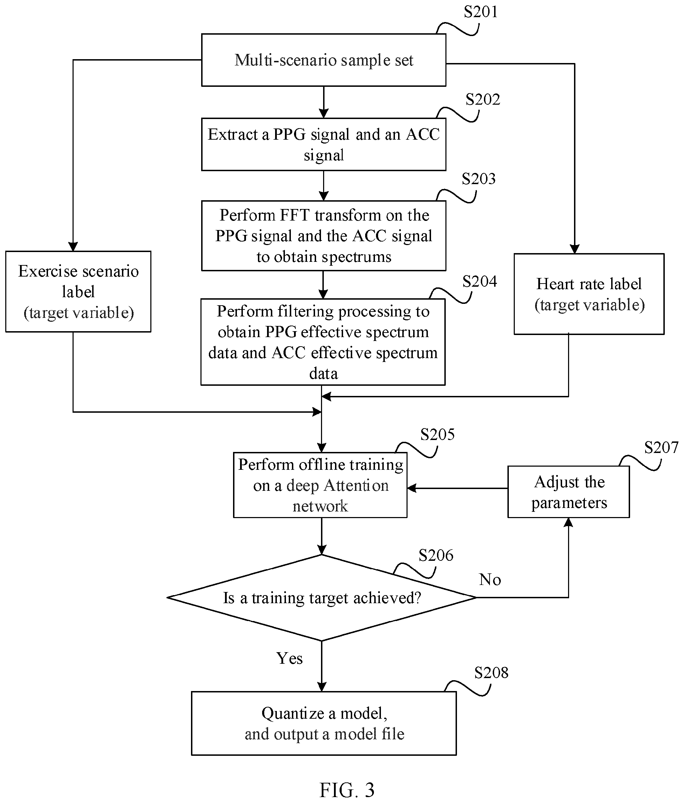

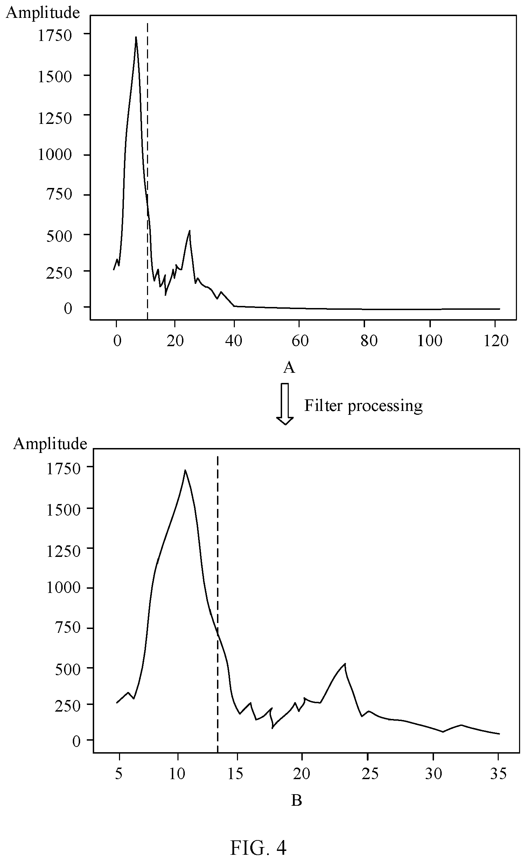

The following separately describes an offline training procedure and an online prediction procedure of the first prediction model in the first embodiment with reference to FIG. 3 and FIG. 4.

FIG. 3 is an offline training procedure of the deep Attention network of the first prediction model according to the first embodiment. As shown in FIG. 3, the offline training procedure of the deep Attention includes steps S201-S208.

S201: Obtain a multi-scenario sample set.

First, a plurality of exercise scenarios that need to be collected may be formulated, for example, walking, running, swimming, climbing, rope jumping, playing football, and playing basketball. It may be understood that the exercise scenarios are examples listed herein. The plurality of exercise scenarios that need to be collected in this embodiment of this application may not be limited thereto, and may be specifically determined based on an actual use requirement. This is not limited in this embodiment of this application.

It should be noted that the exercise scenario herein may cover a resting scenario.

Then, both a wristband device and a heart rate band device are connected, through Bluetooth, to an Android application package (Android application package, APK) that collects data.

Further, when a subject wears the wristband device and the heart rate band device and exercises in a specific scenario, data in the corresponding scenario is collected.

For example, it is assumed that exercise scenario label data is set to running. When the subject runs, data in the running scenario may be collected.

For example, it is assumed that exercise scenario label data is set to walking. When the subject walks, data in the walking scenario may be collected.

For example, it is assumed that exercise scenario label data is set to rope jumping. When the subject jumps rope, data in the rope jumping scenario may be collected.

For example, it is assumed that exercise scenario label data is set to resting. When the subject does not do any exercise, data in the resting scenario may be collected.

When the subject exercises in a specific scenario, the heart rate band device collects heart rate label data in the specific scenario, where the heart rate label data may be used as a target value of model training; and the wristband device collects a PPG signal and an ACC signal in the specific scenario as to-be-trained model parameters.

Then, data collection stops after collection time and a specified collection requirement are met.

For example, when a wearable device detects that a user is in an exercise state, the wearable device may use a sampling frequency of 100 Hz, that is, collect 100 points per second. Alternatively, when the wearable device detects that the user is in a resting state, the wearable device may use a sampling frequency of 25 Hz, that is, collect 25 points per second.

Optionally, duration of a sampling time window may be 8 seconds, 10 seconds, 12 seconds, or any other duration that meets a requirement. This may be specifically set based on an actual use requirement, and is not limited in this embodiment of this application.

For example, a sampling frequency is 25 Hz and time window duration is 12 seconds. If 25 points are collected per second, a total of 300 points can be sampled in 12 seconds.

S202: Sort out all collected sample data to obtain a plurality of channels of PPG signals, three channels of ACC sensor signals, heart rate label data, and exercise scenario label data, to form an offline data set.

It can be learned through comparison between the solution in this application and a conventional algorithm that, in the conventional algorithm, many empirical features need to be manually extracted to train a model; however, in the solution in this application, end-to-end learning can be performed during model training, and data extraction is adaptively implemented in different scenarios, so that model training is more intelligent.

S203: Separately perform FFT transform on the PPG signal and the ACC signal to obtain a spectrum of the PPG signal and a spectrum of the ACC signal.

S204: Separately perform band-pass filtering processing on the spectrum of the PPG signal and the spectrum of the ACC signal, to obtain PPG effective spectrum data and ACC effective spectrum data.

For example, it is assumed that [0.7 HZ, 4 HZ] is a common frequency domain range used for heart rate detection. In this case, when filtering processing is separately performed on the spectrum of the PPG signal and the spectrum of the ACC signal, noise data outside [0.7 HZ, 4 HZ] may be filtered out, so that a frequency domain range useful for heart rate detection can be determined. Spectrum data in the frequency domain range is referred to as effective spectrum data.

Assuming that a human heart rate value is usually 60 bpm, a heart rate value range corresponding to the common frequency domain range [0.7 HZ, 4 HZ] may be [42 bpm, 240 bpm]. The range may be considered as a reference heart rate value range.

A sequence of performing band-pass filtering and FFT transform may not be limited in this embodiment of this application. For example, FFT transform may be performed before band-pass filtering. Certainly, band-pass filtering may be performed before FFT transform.

For example, it is still assumed that a sampling frequency is 25 Hz and time window duration is 12 seconds. In this case, 25 points are collected per second, and a total of 300 points can be sampled in 12 seconds. Through filtering processing, [0, 127] sampling points may be obtained.

Correspondingly, for the 300 points obtained through sampling, 128 effective frequency points may be obtained through FFT transform.

It should be noted that a filter used in this embodiment of this application is not limited, and may be specifically determined based on an actual use requirement.

For ease of description, the PPG signal and the ACC signal that are collected are collectively referred to as time domain sensor data below, and data obtained after the PPG signal and the ACC signal undergo FFT transform and filtering processing is collectively referred to as frequency domain sensor data.

For example, FIG. 4 is a diagram of a waveform of filtering processing performed on frequency domain sensor data, where a vertical coordinate represents an FFT amplitude, and a horizontal coordinate represents a quantity of samples. Assuming that a sampling frequency is 25 Hz, correspondingly, resolution of FFT per horizontal coordinate interval is 25/256=0.097 HZ.

As shown in Ain FIG. 4, in an original spectrum diagram, a horizontal coordinate point range of FFT is [0, 127]. Because the resolution of FFT per horizontal coordinate interval is 0.097 HZ, a frequency interval corresponding to the horizontal coordinate point range [0, 127] of FFT is [0 HZ, 127*0.097 HZ], which is approximately [0 HZ, 12 HZ]. In other words, the original spectrum diagram includes spectrum data in a frequency range [0, 12 Hz], and the spectrum data includes noise data.

Filtering processing is performed on the original spectrum diagram to filter out the noise data. As shown in B in FIG. 4, in a filtered spectrum diagram, a horizontal coordinate point range of FFT is [5, 35]. Because the resolution of FFT per horizontal coordinate interval is 0.097 HZ, a frequency interval corresponding to the horizontal coordinate point range [5, 35] of FFT is [5*0.097 HZ, 35*0.097 HZ], which is approximately [0.5 HZ, 3.5 HZ]. The processed spectrum diagram includes spectrum data in a frequency range [0.7 Hz, 3.5 Hz], and frequency domain data obtained in this case is considered as effective spectrum data.

In this way, effective spectrum data from which noise data is filtered out is used for model training, which helps cancel interference of motion artifact noise to a PPG signal.

S205: Train a deep Attention network by using extracted frequency domain sensor data as a model input and using heart rate information and exercise scenario information as model outputs.

The PPG effective spectrum data and the ACC effective spectrum data may be used as inputs of the deep Attention network, and the heart rate label data and the exercise scenario label data may be used as target values, to perform offline training on the deep Attention network.

In this embodiment of this application, because motion artifact noise is distributed differently in different scenarios, in the solution in this application, end-to-end training is performed on the deep Attention network, so that both a scenario identification capability and a heart rate detection capability can be obtained, to fit a nonlinear relationship between a scenario, noise, and a heart rate to a greater extent.

S206: Determine whether a training target is achieved.

If the training target is not achieved, step S207 continues to be performed; or if the training target is achieved, step S208 continues to be performed.

S207: Adjust, through cross-validation, the parameters in S205 that are used for model training, to constantly optimize the model.

The model may be optimized by adjusting a weight and a deviation of a network (for example, a convolutional neuron).

In this embodiment of this application, when the training target is not achieved, the parameter is constantly adjusted to optimize the model until the training target is achieved, so that the model can learn label information in a complex scenario and an adaptive weight parameter of the Attention network, to achieve effects of signal denoising, signal fusion, and complex scenario identification in a plurality of complex scenarios.

It can be learned through comparison between the solution in this application and a conventional algorithm that, processing such as adaptive filtering, principal component analysis, and signal decomposition that are used in the conventional heart rate prediction algorithm is applicable to only a specific noise scenario, and as a model algorithm with a fixed parameter, the conventional heart rate prediction algorithm cannot adaptively predict a heart rate value in a complex scenario; however, in the solution in this application, an Attention mechanism can be used to perform adaptive fitting between a signal feature in a scenario and noise, adaptively adjust a network weight parameter, and process motion artifacts in each scenario, thereby improving a generalization capability.

S208: Quantize the model, and output a model file.

In this embodiment of this application, offline training is performed on the deep Attention network to obtain a deep Attention model. Output tasks of the model are a heart rate value and scenario information. Therefore, the deep Attention model may also be referred to as a scenario identification and heart rate detection model.

In this embodiment of this application, a trained model may be quantized by using a parameter of 8 Bits or 16 Bits, and network structure information and quantized parameter data of the trained model are stored. After the model is quantized, storage space of the wearable device is reduced, and a speed of calculation of an integer type is increased. Therefore, storage space of a component of the wearable device can be reduced, and a calculation speed can be increased.

In this embodiment of this application, the deep Attention network performs learning by using an attention mechanism, and different noise cancellation operations are performed based on the scenario information, so that the deep Attention network model obtained through training is a dynamic adaptive model, which can perform end-to-end learning, and can adaptively implement data denoising processing and feature extraction in different scenarios, to achieve effects of signal denoising, signal fusion, and complex scenario identification in a plurality of complex scenarios. Therefore, when heart rate detection is performed by using the deep Attention network model obtained through training, precision of heart rate detection can be improved.

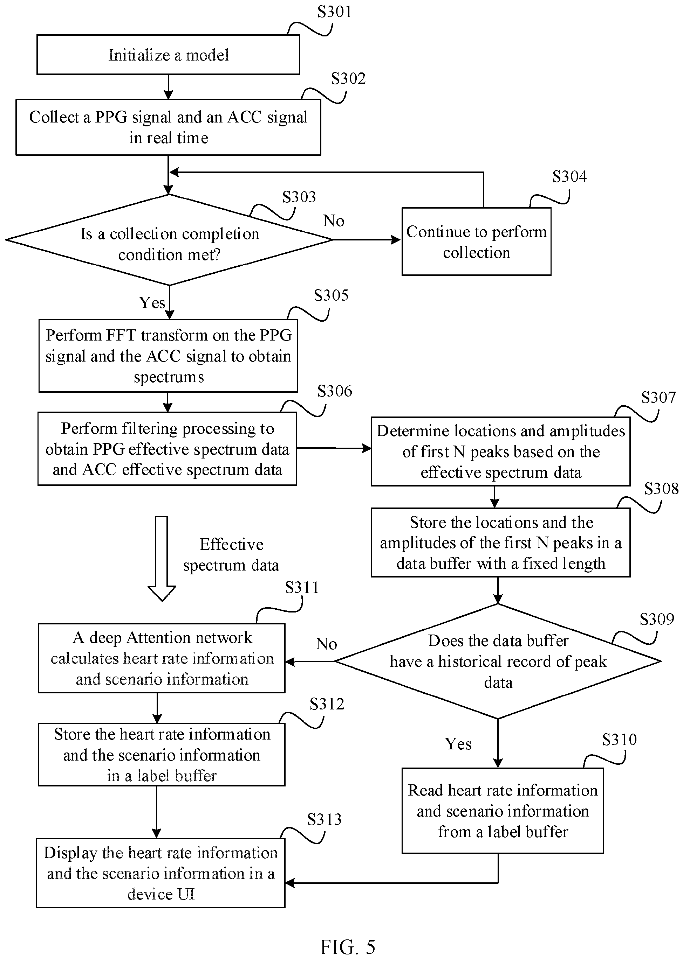

With reference to FIG. 3, the foregoing describes in detail the procedure for obtaining the scenario identification and heart rate detection model by performing offline training on the deep Attention network. With reference to FIG. 5, the following describes in detail a procedure for performing online prediction by using the scenario identification and heart rate detection model obtained through training. As shown in FIG. 5, the online prediction procedure includes the following steps S301-S313.

S301: Initialize a model.

A wearable device starts a heart rate detection module, loads a prestored structure and parameter of the deep Attention model, to establish a forward inference procedure.

S302: Collect a PPG signal and an ACC signal in real time.

The wearable device collects the synchronous and real-time PPG signal and ACC signal.

S303: Determine whether a collection completion condition is met.

Optionally, when an amount of collected data reaches a preset amount, it can be considered that the collection completion condition is met.

Optionally, when data collection duration reaches preset duration, it can be considered that the collection completion condition is met.

For example, when the PPG signal and the ACC signal start to be collected, a timer is started, and duration of the timer may be set to the preset duration. Once the timer expires, it is considered that data collection is completed, and the collection completion condition is met.

After the collection completion condition is met, the wearable device proceeds to a next step S305 of the procedure; or if the collection completion condition is not met, step S304 is performed to continue to collect the PPG signal and the ACC signal until the collection completion condition is met.

S305: Separately perform FFT transform on the collected PPG signal and ACC signal to obtain a spectrum of the PPG signal and a spectrum of the ACC signal.

S306: Separately perform band-pass filtering processing on the spectrum of the PPG signal and the spectrum of the ACC signal to obtain PPG effective spectrum data and ACC effective spectrum data.

S307: Determine frequency domain locations and amplitudes of first N highest peaks based on the PPG effective spectrum data and the ACC effective spectrum data.

For example, when N is 3, the wearable device may determine frequency domain locations and amplitudes of first three highest peaks of the PPG effective spectrum data and frequency domain locations and amplitudes of first three highest peaks of the ACC effective spectrum data.

In frequency domain locations and amplitudes of first N highest peaks of frequency domain sensor data, frequency domain locations and amplitudes of PPG and ACC spectrum peaks may be represented in an array form, for example, [Fₚ, Aₚ, Fₐ, Aₐ], which is referred to as a peak array below, where Fₚ and Aₚ respectively represent the frequency domain location and the amplitude of the PPG spectrum peak, and Fₐ and Aₐ respectively represent the frequency domain location and the amplitude of the ACC spectrum peak.

Through the deep heart rate detection model obtained through training in this embodiment of this application, calculation power consumption of the wearable device can be reduced with reference to a data record of first three peaks of FFT and a comparison mechanism.

S308: Store, in a data buffer, the frequency domain locations and the amplitudes of the first N highest peaks of the frequency domain sensor data.

In this embodiment of this application, a data buffer (Data_Buffer) and a label buffer (Label_Buffer) may be disposed on the wearable device.

The data buffer may be a first-in first-out buffer with a specific length, and may be configured to store a specific quantity of peak arrays. The label buffer may be configured to store a heart rate value obtained by performing heart rate detection by using the model.

S309: Determine whether a current peak array has appeared in the data buffer.

In an aspect, if the current peak array has appeared in the data buffer, it indicates that a heart rate value has been obtained in advance by performing heart rate prediction by using the model. To be specific, the current peak array has a corresponding heart rate value, and the corresponding heart rate value is stored in the label buffer. In this case, the following step S310 continues to be performed.

In another aspect, if the current peak array has not appeared in the data buffer, it indicates that the current peak array has no corresponding heart rate value, and heart rate prediction needs to be performed by using the model. Therefore, the following step S311 continues to be performed, that is, forward inference is performed by using the deep Attention network model to calculate a heart rate value.

In some possible implementations, first, heart rate monitoring is performed in preset duration (for example, 12 seconds), heart rate monitoring data is stored in the data memory, and a corresponding heart rate monitoring result is stored in the label memory. Then, in a subsequent heart rate monitoring process, the determining action in S309 may be performed, and currently obtained heart rate monitoring data is compared with the heart rate monitoring data stored in the data memory, to determine whether the currently obtained heart rate monitoring data has appeared in the data buffer.

According to the foregoing solution, history review is performed by using the data buffer, so that repeated calculation can be avoided, and calculation power consumption of the wearable device can be reduced.

S310: When it is determined in S309 that the current peak array has appeared in the data buffer, read a corresponding heart rate and corresponding scenario information that are stored in the label memory.

After S310 is performed, the following step S313 continues to be performed.

S311: When it is determined in S309 that the current peak array has not appeared in the data buffer, the deep Attention model performs forward inference by using the PPG effective spectrum data and the ACC effective spectrum data as inputs of the deep Attention model, to calculate corresponding heart rate information and scenario information.

Through the deep heart rate detection model obtained through training in this embodiment of this application, a calculation speed can be increased in a real-time prediction phase by using the simplified quantized model.

S312: Store the heart rate information and the scenario information in the label memory.

A function of storing the heart rate value and the scenario information in the label memory is as follows: When it is determined that a peak array has appeared in the data buffer, it indicates that a heart rate value has been obtained in advance by performing heart rate prediction by using the model, and the label memory has stored corresponding heart rate information and scenario information. Therefore, the corresponding heart rate value and scenario information stored in the label memory can be directly read. In this case, a current scenario does not need to be identified by using a scenario identification model, and a heart rate value does not need to be detected by using a heart rate detection model. In this way, repeated calculation can be avoided, and calculation power consumption of the wearable device can be reduced.

S313: Transmit the heart rate information and the scenario information to a user interface (user interface, UI) of the wearable device for presentation.

In this embodiment of this application, the wearable device may perform a monitoring operation such as heart rate warning, and transmit the scenario information to another component of the wearable device. In this way, power consumption of the wearable device and an intelligent light adjustment algorithm of the wearable device can be adjusted based on an actual use requirement.

It can be learned through comparison between the solution in this application and a conventional algorithm that, the conventional heart rate detection algorithm has no function of simultaneously performing heart rate detection and scenario identification; however, in the solution in this application, it is considered that there is a strong correlation between a scenario and a heart rate, and if these relationships are ignored, a model identification capability is low. Therefore, in this embodiment of this application, a deep Attention learning mechanism is used, so that a plurality of exercise scenarios can be identified, corresponding adaptive fitting between a scenario and noise can be performed, and excessive data preprocessing and manual experience parameter formulation are not required.

The foregoing describes the deep learning-based heart rate detection method provided in the first embodiment, and the deep Attention model training procedure and the online prediction procedure that correspond to the method. The following further describes a deep learning-based heart rate detection method provided in a second embodiment, and a model training procedure and an online prediction procedure that correspond to the method.

### Second Embodiment

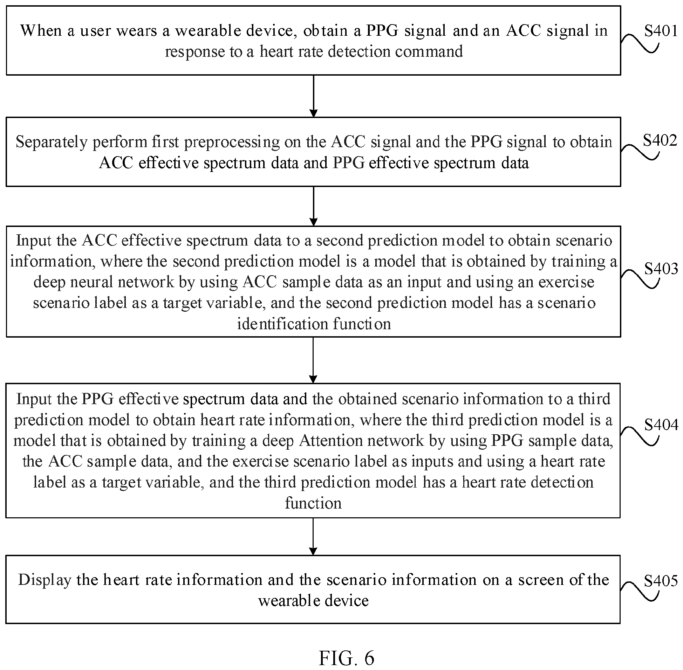

With reference to FIG. 6, the following describes in detail the deep learning-based heart rate detection method according to the second embodiment of this application.

FIG. 6 is a schematic flowchart of the heart rate detection method according to the second embodiment of this application. As shown in FIG. 6, the method includes the following steps S401-S405.

S401: When a user wears a wearable device, obtain a PPG signal and an ACC signal in response to a heart rate detection command.

S402: Separately perform first preprocessing on the ACC signal and the PPG signal to obtain ACC effective spectrum data and PPG effective spectrum data.

For detailed descriptions of S401 and S402, refer to the detailed descriptions of S101 and S102 in the first embodiment. Details are not described herein again.

S403: Input the ACC effective spectrum data to a second prediction model to obtain scenario information, where the second prediction model is a model that is obtained by training a deep neural network by using ACC sample data as an input and using an exercise scenario label as a target variable, and the second prediction model has a scenario identification function.

In this embodiment of this application, the wearable device may use the ACC effective spectrum data as an input parameter of the second prediction model to perform an online prediction procedure by using the second prediction model. Through the second prediction model (which may also be referred to as a scenario identification model), the wearable device may identify whether a current exercise scenario of the user is resting or exercising, and may precisely identify a specific type of exercise, for example, walking, swimming, running, or climbing.

Similarly, for ease of description, herein, the exercise scenario identified by the wearable device is still described as first scenario information.

S404: Input the PPG effective spectrum data and the obtained scenario information to a third prediction model to obtain heart rate information, where the third prediction model is a model that is obtained by training a deep Attention network by using PPG sample data, the ACC sample data, and the exercise scenario label as inputs and using a heart rate label as a target variable, and the third prediction model has a heart rate detection function.

In this embodiment of this application, after identifying the exercise scenario, the wearable device may use the identified scenario information and the PPG effective spectrum data as input parameters of the third prediction model to perform an online prediction procedure by using the third prediction model. Through the third prediction model (which may also be referred to as a heart rate detection model), the wearable device may accurately detect a heart rate value of the user in a current exercise scenario.

For ease of description, herein, the heart rate value detected by the wearable device is still described as the heart rate information. In this way, after the online prediction procedures respectively performed by using the second prediction model and the third prediction model end, the scenario information and the heart rate information may be output.

It should be noted that, the third prediction model provided in this embodiment of this application is a scenario identification and heart rate detection model that is obtained by training the deep Attention network based on the heart rate information and the exercise scenario information. To be specific, in the solution in this application, the exercise scenario information and the heart rate information are combined to perform model training, so that exercise noise can be reduced, to avoid impact of motion artifacts on heart rate detection accuracy. Therefore, a heart rate value output by the third prediction model is more precise.

An offline training procedure of the second prediction model (namely, the scenario identification model) and an offline training procedure of the third prediction model (namely, the heart rate detection model) are described below in detail. Details are not described herein.

S405: Display the heart rate information and the scenario information.

For detailed descriptions of S405, refer to the detailed descriptions of S104 in the first embodiment. Details are not described herein again.

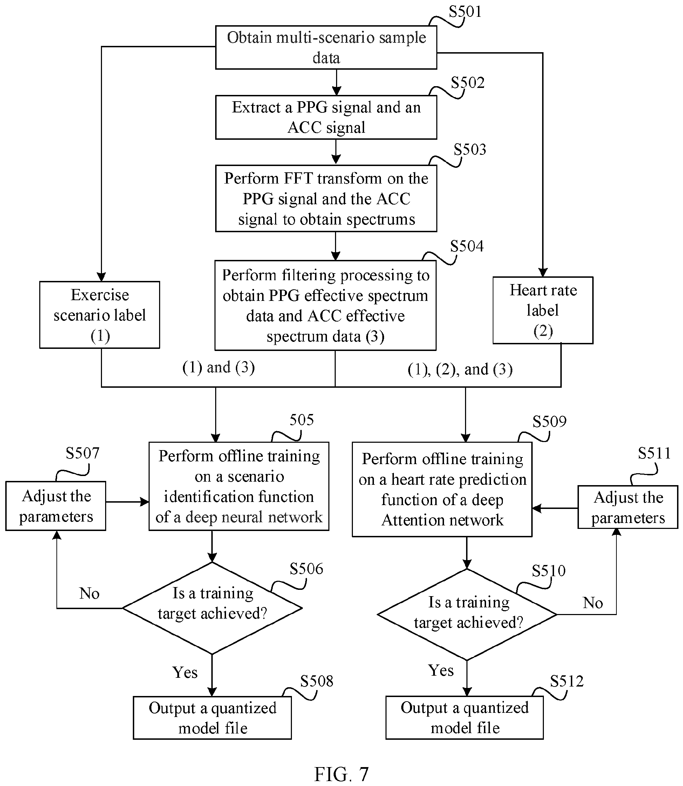

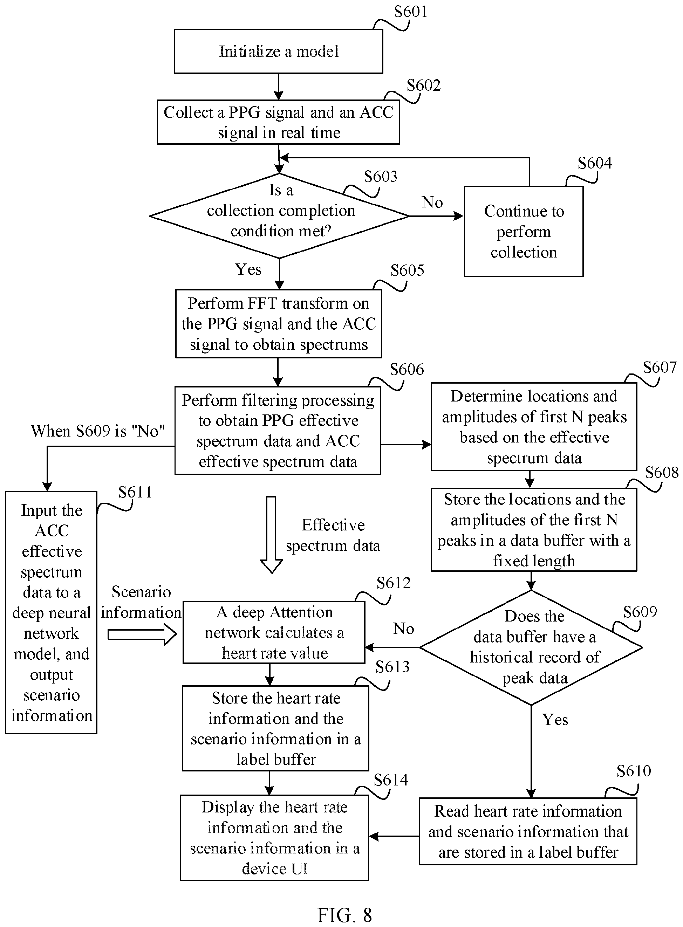

The following respectively describes an offline model training procedure and an online prediction procedure in the second embodiment with reference to FIG. 7 and FIG. 8.

FIG. 7 shows an offline training procedure of a scenario identification model and a heart rate detection model in the second embodiment. As shown in FIG. 7, the offline model training procedure includes steps S501-S512. Offline training is performed on a deep neural network to obtain the scenario identification model, and offline training is performed on an Attention network to obtain the heart rate detection model.

S501: Obtain multi-scenario sample data.

The multi-scenario sample data is also referred to as a multi-scenario sample set.

S502: Sort out all collected sample data to obtain a plurality of channels of PPG signals, three channels of ACC sensor signals, heart rate label data, and scenario label data, to form an offline data set.

S503: Separately perform FFT transform on the PPG signal and the ACC signal to obtain a spectrum of the PPG signal and a spectrum of the ACC signal.

S504: Perform filtering processing on the spectrum of the PPG signal and the spectrum of the ACC signal to filter out noise data outside [0.7 HZ, 4 HZ], to obtain PPG effective spectrum data and ACC effective spectrum data.

In addition, the exercise scenario label data and the heart rate label data are obtained from the multi-scenario sample data.

For detailed descriptions of 5501-5504, refer to the detailed descriptions of S201-S204 in the first embodiment. Details are not described herein again.

S505: Train a deep neural network by using the PPG effective spectrum data and the ACC effective spectrum data (3) as inputs of the deep neural network and using the exercise label data (1) as a target value, that is, using exercise scenario information as an output, so that the deep neural network learns how to identify multi-scenario information.

In this embodiment of this application, a deep Attention learning mechanism is used, so that a plurality of exercise scenarios can be identified, corresponding adaptive fitting between a scenario and noise can be performed, and excessive data preprocessing and manual experience parameter formulation are not required.

S506: Determine whether a training target is achieved.

If the training target is not achieved, step S507 continues to be performed; or if the training target is achieved, step S508 continues to be performed.

S507: Adjust, through cross-validation, the parameters in S505 that are used for model training, to constantly optimize the model.

In this embodiment of this application, when the training target is not achieved, the parameter of the model may be adjusted through cross-validation to constantly optimize the model, until the training target is achieved, so that the deep neural network learns how to identify multi-scenario information, to achieve effects of signal denoising, signal fusion, and complex scenario identification in a plurality of complex scenarios.

S508: Quantize the model, and output a model file.

In this embodiment of this application, offline training is performed on the deep neural network to obtain a deep neural network model. An output task of the model is exercise scenario information. Therefore, the deep neural network model may also be referred to as a scenario identification model.

A trained model may be quantized by using a parameter of 8 Bits or 16 Bits, and network structure information and quantized parameter data of the trained model are stored. The operation can reduce storage space of a component of a wearable device and increase a calculation speed.

The scenario identification model is obtained above through training by using steps S505-S508, and the heart rate detection model is obtained below through training by using steps S509-S512.

S509: Use the exercise scenario label data (1), and the PPG effective spectrum data and the ACC effective spectrum data (3) as inputs of a deep Attention network, and use the heart rate label data (3) as a target value of training, that is, use heart rate information as an output, to instruct the Attention network to learn to perform nonlinear adaptive learning on the exercise scenario data and the frequency domain sensor data, to train a heart rate detection capability of the Attention network.

In this embodiment of this application, because motion artifact noise is distributed differently in different scenarios, in the solution in this application, end-to-end training is performed the deep Attention network to fit a nonlinear relationship between a scenario, noise, and a heart rate to a greater extent, thereby improving precision of heart rate detection in different scenarios.

S510: Determine whether a training target is achieved.

If the training target is not achieved, step S511 continues to be performed; or if the training target is achieved, step S512 continues to be performed.

S511: Adjust, through cross-validation, the parameters in S510 that are used for model training, to constantly optimize the model.

The model may be optimized by adjusting a weight and a deviation of a network (for example, a convolutional neuron).

In this embodiment of this application, when the training target is not achieved, the parameter of the model may be adjusted through cross-validation to constantly optimize the model, until the training target is achieved, so that the deep Attention network learns to detect heart rate values in different exercise scenarios.

According to the solution in this application, an Attention mechanism can be used to perform adaptive fitting between a signal feature in a scenario and noise, adaptively adjust a network weight parameter, and process motion artifacts in each scenario, thereby improving a generalization capability.

S512: Quantize the model, and output a model file.

In this embodiment of this application, offline training is performed on the deep Attention network to obtain a deep Attention model. An output task of the model is a heart rate value. Therefore, the deep Attention model may also be referred to as a heart rate detection model.

A trained model may be quantized by using a parameter of 8 Bits or 16 Bits, and network structure information and quantized parameter data of the trained model are stored. After the model is quantized, storage space of the wearable device is reduced, and a speed of calculation of an integer type is increased. Therefore, the operation can reduce storage space of a component of the wearable device and increase a calculation speed.

The following compares the offline model training procedure in the first embodiment with the offline model training procedure in the second embodiment, and a difference between the two procedures lies in the following.

In the first embodiment, the Attention network model obtained through training by using the frequency domain sensor signal as an input of the Attention network and using the exercise scenario information and the heart rate information as outputs has the scenario identification function and the heart rate detection function.

In the second embodiment, the Attention network model obtained through training by using the frequency domain sensor data and the exercise scenario label data as inputs of the Attention network and using the heart rate information as an output has the heart rate detection function.

With reference to FIG. 7, the foregoing describes in detail the procedure for performing offline training on the deep neural network to obtain the scenario identification model and the procedure for performing offline training on the deep Attention network to obtain the heart rate detection model. With reference to FIG. 8, the following describes in detail a procedure for performing online prediction by using the deep neural network and the Attention network that are obtained through training. As shown in FIG. 8, the online prediction procedure includes the following steps S601-S614.

S601: Initialize a model.

A wearable device starts a heart rate detection module, loads prestored structures and parameters of a deep neural network model and a deep Attention model, to establish a forward inference procedure.

S602: Collect a PPG signal and an ACC signal in real time.

S603: Determine whether a collection completion condition is met.

The wearable device collects the synchronous and real-time PPG signal and ACC signal. After the collection completion condition is met, the wearable device proceeds to a next step S605 of the procedure; or if the collection completion condition is not met, step S604 is performed to continue to collect the PPG signal and the ACC signal until the collection completion condition is met.

S605: Separately perform FFT transform on the collected PPG signal and ACC signal to obtain a spectrum of the PPG signal and a spectrum of the ACC signal.

S606: Separately perform band-pass filtering on the frequency domain PPG signal and the frequency domain ACC signal to obtain PPG effective spectrum data and ACC effective spectrum data.

A sequence of performing band-pass filtering and FFT transform may not be limited in this embodiment of this application. For example, FFT transform may be performed before band-pass filtering. Certainly, band-pass filtering may be performed before FFT transform.

S607: Determine frequency domain locations and amplitudes of first N highest peaks of frequency domain sensor data.

The frequency domain sensor data includes the PPG effective spectrum data and the ACC effective spectrum data.

N may be a positive integer. For example, when N is 3, the wearable device may determine frequency domain locations and amplitudes of first three highest peaks of the PPG effective spectrum data and frequency domain locations and amplitudes of first three highest peaks of the ACC effective spectrum data.

In the frequency domain locations and the amplitudes of the first N highest peaks of the frequency domain sensor data, frequency domain locations and amplitudes of PPG and ACC spectrum peaks may be represented in an array form, for example, [Fₚ, Aₚ, Fₐ, Aₐ], which is referred to as a peak array below.

Through the deep heart rate detection model obtained through training in this embodiment of this application, calculation power consumption of the wearable device can be reduced with reference to recording of first N-peak data of FFT and a comparison mechanism.

S608: Store, in a data buffer, the frequency domain locations and the amplitudes of the first N highest peaks of the frequency domain sensor data.

In this embodiment of this application, a data buffer and a label buffer may be disposed on the wearable device.

The data buffer may be a first-in first-out buffer with a specific length, and may be configured to store a specific quantity of peak arrays. The label buffer may be configured to store a heart rate value obtained by performing heart rate detection by using the model.

S609: Determine whether a current peak array has appeared in the data buffer.

In an aspect, if the current peak array has appeared in the data buffer, it indicates that a heart rate value has been obtained in advance by performing heart rate prediction by using the model. To be specific, the current peak array has a corresponding heart rate value, and the corresponding heart rate value is stored in the label buffer. In this case, the following step S610 continues to be performed.

In another aspect, if the current peak array has not appeared in the data buffer, it indicates that the current peak array has no corresponding heart rate value, and heart rate prediction needs to be performed by using the model. Therefore, the following step S611 continues to be performed, that is, forward inference is performed by using the deep Attention network model to calculate a heart rate value.

S611: When it is determined in S609 that the current data has not appeared in the data buffer, use the PPG effective spectrum data and the ACC effective spectrum data as inputs of a neural network model to identify current exercise scenario information by using the deep neural network model, and transmit the exercise scenario information to the deep Attention model.

S612: A deep Attention model performs forward inference by using the identified exercise scenario information, the PPG effective spectrum data, and the ACC effective spectrum data as inputs of the deep Attention model, to calculate a heart rate value in a current scenario.

Through the deep heart rate detection model obtained through training in this embodiment of this application, a calculation speed can be increased in a real-time prediction phase by using the simplified quantized model.

S613: Store the heart rate value and the scenario information in the label memory.

A function of storing the heart rate value and the scenario information in the label memory is as follows: When it is determined that a peak array has appeared in the data buffer, it indicates that a heart rate value has been obtained in advance by performing heart rate prediction by using the model, and the label memory has stored a corresponding heart rate value and corresponding scenario information. Therefore, the corresponding heart rate value and scenario information stored in the label memory can be directly read. In this case, a current scenario does not need to be identified by using a scenario identification model, and a heart rate value does not need to be detected by using a heart rate detection model.

S614: Transmit the heart rate value and the scenario information to a UI interface of the wearable device for presentation.

S610: When it is determined in S609 that the current peak array has appeared in the data buffer, read a corresponding heart rate value and corresponding scenario information that are stored in the label memory.

According to the foregoing solution, history review is performed by using the data buffer, so that repeated calculation can be avoided, and calculation power consumption of the wearable device can be reduced.

After S610 is performed, step S614 continues to be performed, that is, the heart rate value and the scenario information are transmitted to the UI interface of the wearable device for presentation.

In this embodiment of this application, the wearable device may perform a monitoring operation such as heart rate warning, and transmit the scenario information to another component of the wearable device. In this way, power consumption of the wearable device and an intelligent light adjustment algorithm of the wearable device can be adjusted based on an actual use requirement.

The following compares the online model prediction procedure in the second embodiment with the online model prediction procedure in the first embodiment, and a difference between the two procedures lies in the following.

In the first embodiment, the PPG frequency domain data and the ACC frequency domain data are used as inputs of the deep Attention model, and the deep Attention model outputs both the exercise scenario information and the heart rate information. The model has the scenario identification function and the heart rate detection function.

In the second embodiment, the scenario identification function and the heart rate detection function are separated. Scenario information identification is first performed by using the deep neural network model, and then a scenario identification result and frequency domain sensor data are used as inputs of the deep Attention model, so that the deep Attention model outputs the heart rate value.

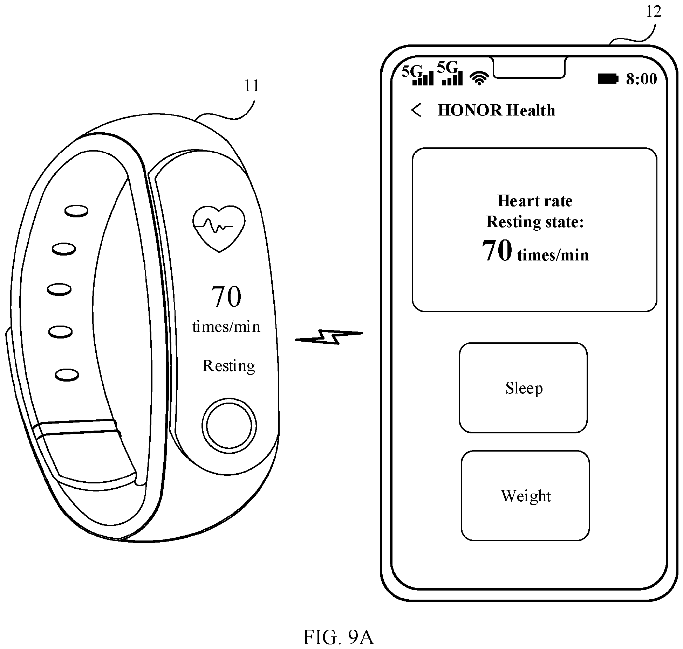

FIG. 9A to FIG. 9C are schematic diagrams of interfaces applied to a deep learning-based heart rate detection method according to an embodiment of this application.

As shown in FIG. 9A, when a wearable device 11 detects that a user wearing the wearable device 11 is in a resting state, the wearable device 11 performs heart rate detection and displays a detection result: 70 times/minute, and resting. It can be learned that the heart rate detection result in the solution in this application may include a heart rate and current scenario information. When the wearable device 11 maintains a wireless connection to a terminal device 12, the wearable device 11 may send the heart rate detection result to the terminal device 12, and the terminal device 12 displays the heart rate detection result for the user to view on the terminal device 12.

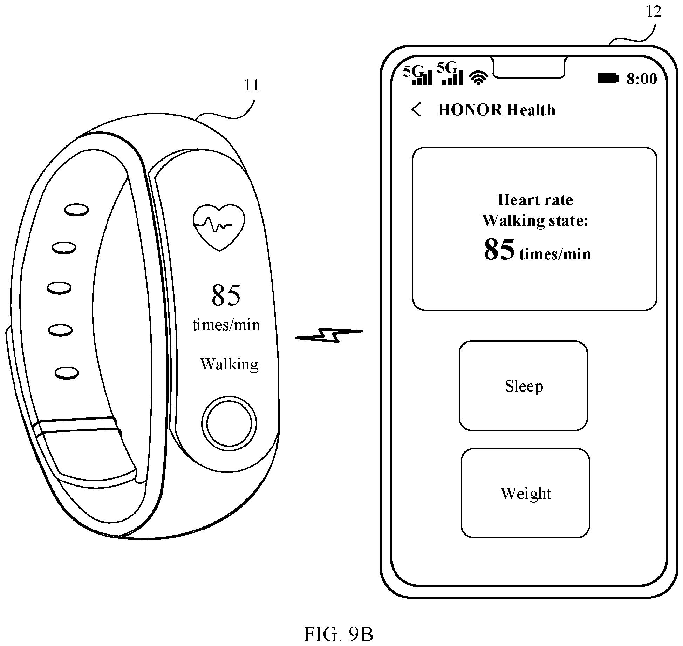

As shown in FIG. 9B, when the wearable device 11 detects that the user is in an exercise state of walking, the wearable device 11 performs heart rate detection and displays a detection result: 85 times/minute, and walking. It can be learned that the heart rate detection result in the solution in this application may include a heart rate and current scenario information. When the wearable device 11 maintains a wireless connection to the terminal device 12, the wearable device 11 may send the heart rate detection result to the terminal device 12, and the terminal device 12 displays the heart rate detection result for the user to view on the terminal device 12.

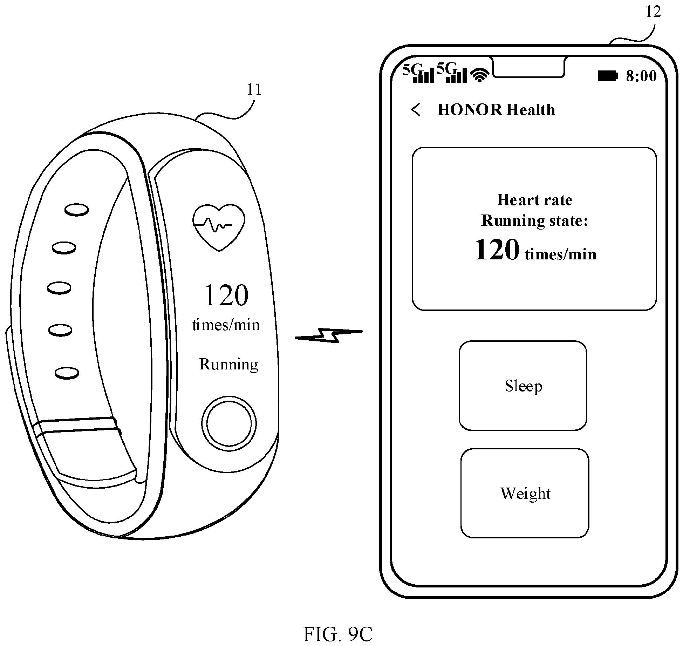

As shown in FIG. 9C, when the wearable device 11 detects that the user is in an exercise state of running, the wearable device 11 performs heart rate detection and displays a detection result: 120 times/minute, and resting. It can be learned that the heart rate detection result in the solution in this application may include a heart rate and current scenario information. When the wearable device 11 maintains a wireless connection to the terminal device 12, the wearable device 11 may send the heart rate detection result to the terminal device 12, and the terminal device 12 displays the heart rate detection result for the user to view on the terminal device 12.

In conclusion, the solutions in this application can achieve the following technical effects.

In an aspect, the deep Attention network learns, by using an attention mechanism, to perform different noise cancellation operations based on scenario information, and is a dynamic adaptive model, which can perform end-to-end learning, and can adaptively implement data denoising processing and feature extraction in different scenarios.

In another aspect, the deep Attention learning mechanism can be used to identify a plurality of exercise scenarios, and perform corresponding adaptive fitting between a scenario and noise, so that excessive data preprocessing and manual experience parameter formulation are not required.

In still another aspect, through the deep heart rate detection model provided in the embodiments of this application, a calculation speed in a real-time prediction phase can be increased by using the simplified quantized model, and calculation power consumption of the wearable device can be reduced with reference to a data record of first three peaks of FFT and a comparison mechanism.

It should be noted that the solutions provided in the embodiments of this application may be further applied to another scenario such as blood pressure estimation or blood oxygen concentration calculation. A specific implementation of performing blood pressure estimation or blood oxygen concentration calculation is similar to the implementation of performing heart rate detection by using the deep Attention network model in the first embodiment and the second embodiment.

It should also be noted that in the embodiments of this application, "greater than" may be replaced with "greater than or equal to", and "less than or equal to" may be replaced with "less than"; or "greater than or equal to" may be replaced with "greater than", and "less than" may be replaced with "less than or equal to".

The embodiments described in this specification may be independent solutions, or may be combined based on internal logic. These solutions all fall within the protection scope of this application.

It may be understood that, the methods and operations implemented by the wearable device in the foregoing method embodiments may also be implemented by a component (for example, a chip or a circuit) that can be used in the wearable device.

The method embodiments provided in this application are described above, and apparatus embodiments provided in this application are described below. It should be understood that description of the apparatus embodiments corresponds to the description of the method embodiments. Therefore, for content that is not described in detail, refer to the foregoing method embodiments. For brevity, details are not described herein again.

The solutions provided in the embodiments of this application are mainly described above from a perspective of a method step. It may be understood that, to implement the foregoing functions, the wearable device implementing the method includes corresponding hardware structures and/or software modules for performing the functions. A person skilled in the art may be aware that the example units and algorithm steps described with reference to the embodiments disclosed in this specification can be implemented in this application by using hardware or a combination of hardware and computer software. Whether a function is performed by hardware or by driving hardware by using computer software depends on particular applications and design constraints of the technical solutions. A skilled person may use different methods to implement the described functions for each specific application, but it should not be considered that the implementation goes beyond the protection scope of this application.

In embodiments of this application, the wearable device may be divided into functional modules based on the foregoing method examples. For example, each functional module may be obtained through division for each corresponding function, or two or more functions may be integrated into one processing module. The integrated module may be implemented in a form of hardware, or may be implemented in a form of a software functional module. It should be noted that in embodiments of this application, module division is an example, and is merely logical function division. In actual implementation, there may be another division manner. An example in which each functional module is obtained through division for each corresponding function is used below for description.

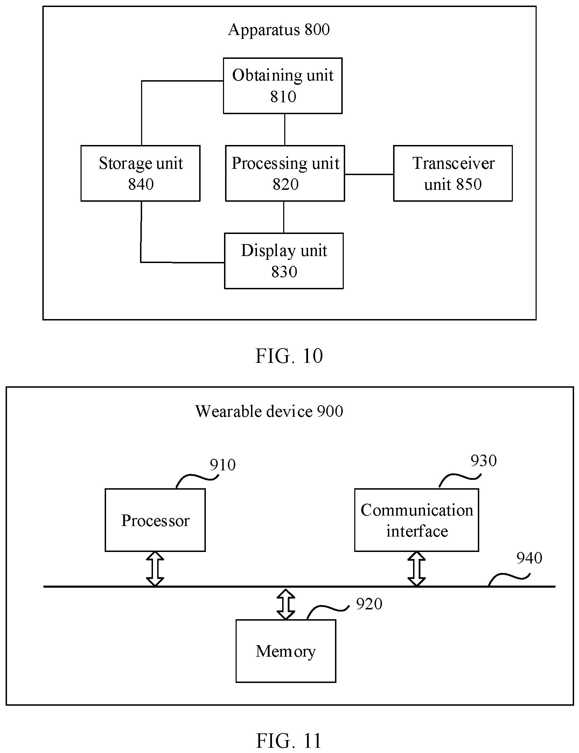

FIG. 10 is a schematic block diagram of a deep learning-based heart rate detection apparatus 800 according to an embodiment of this application. The apparatus 800 may be configured to perform actions performed by the wearable device in the foregoing method embodiments. The apparatus 800 includes an obtaining unit 810, a processing unit 820, and a display unit 830.

The obtaining unit 810 is configured to: when a user wears the wearable device, obtain a photoplethysmography PPG signal and an acceleration ACC signal in response to a heart rate detection command.

The processing unit 820 is configured to obtain heart rate information and scenario information based on ACC effective spectrum data corresponding to the ACC signal, PPG effective spectrum data corresponding to the PPG signal, and a target prediction model.

The display unit 830 is configured to display the heart rate information and the scenario information on a screen of the wearable device.

The target prediction model is a model that is obtained by training a deep attention Attention network by using ACC sample data and PPG sample data as inputs and using a heart rate label and an exercise scenario label as target variables, and the target prediction model has a scenario identification function and a heart rate prediction function.

According to this solution, the deep Attention network is trained by using the PPG and ACC sensor signals as inputs and using the heart rate information and the exercise scenario information as model outputs, and heart rate detection is performed by using a model obtained through training. Because motion artifact noise is distributed differently in different scenarios, a nonlinear relationship between a scenario, noise, and a heart rate may be fitted by using a deep Attention network learning mechanism, so that data denoising processing and feature extraction can be adaptively implemented in different scenarios, to achieve effects of signal denoising, signal fusion, and complex scenario identification in a plurality of complex scenarios, thereby canceling interference of motion artifact noise to a PPG signal, and improving precision of heart rate detection. Therefore, this solution can resolve a problem that a heart rate detection result is not precise because the PPG signal is interfered with by motion artifacts.

In some possible implementations, the apparatus 800 further includes a storage unit 840. For example, the storage unit 840 may include a label buffer and a data buffer. Specifically, the storage unit 840 may be configured to:
when the heart rate information and the scenario information are obtained, record the heart rate information and the scenario information in the label buffer; and
record, in the data buffer, spectrum peak-point data corresponding to the heart rate information and the scenario information.

The spectrum peak-point data corresponding to the heart rate information and the scenario information includes a peak point location and an amplitude that are of a PPG spectrum and a peak point location and an amplitude that are of an ACC spectrum.

According to this solution, a model may be first trained in an initial period of heart rate detection, heart rate information and scenario information obtained by the model are stored, and corresponding peak locations and amplitudes of PPG frequency domain data and ACC frequency domain data are stored. Then, in a later period of heart rate detection, the stored data can be directly invoked when a condition is met. This can save calculation resources, and reduce a delay, so that a heart rate value can be obtained more quickly.

In some possible implementations, the obtaining unit 810 is specifically configured to:
obtain first N pieces of peak-point spectrum data based on the PPG effective spectrum data and the ACC effective spectrum data; and
if no peak-point spectrum data in the first N pieces of peak-point spectrum data is prestored in the data buffer, obtain the heart rate information and the scenario information based on the ACC effective spectrum data corresponding to the ACC signal, the PPG effective spectrum data corresponding to the PPG signal, and the target prediction model; or
if any peak-point spectrum data in the first N pieces of peak-point spectrum data is prestored in the data buffer, read, from the label buffer, heart rate information and scenario information that correspond to the any peak-point spectrum data.

According to this solution, locations and amplitudes of first three highest peaks of the PPG frequency domain data and the ACC frequency domain data may be separately obtained, and whether current data has appeared in the data buffer is checked. If the current data has not appeared, the current data enters the model; or if the current data has appeared, corresponding heart rate information and scenario information stored in the label buffer are read. This can save calculation resources, and reduce a delay, so that a heart rate value can be obtained more quickly.

In some possible implementations, the processing unit 820 is specifically configured to:
inputting the ACC effective spectrum data and the PPG effective spectrum data to a first prediction model to obtain the heart rate information and the scenario information.

The target prediction model is the first prediction model, the first prediction model is a model that is obtained by training the deep attention Attention network by using the ACC sample data and the PPG sample data as inputs and using the heart rate label and the exercise scenario label as target variables, and the first prediction model has the scenario identification function and the heart rate prediction function.

The foregoing solution provides a heart rate detection solution: The PPG frequency domain data and the ACC frequency domain data are used as inputs, and the exercise scenario information and the heart rate information are output. In this way, in actual implementation, the wearable device may output both current scenario information and a current heart rate value when detecting a heart rate. According to the solution in this application, a user behavior or an exercise state is considered, to support detection of heart rate values of the user in different behavior states (for example, a resting state and various exercise states). The solution in this application can resolve a problem of various types of noise interference caused to a PPG signal in different scenarios, to effectively detect a heart rate in different noise scenarios.

In some other possible implementations, the processing unit 820 is specifically configured to:
input the ACC effective spectrum data to a second prediction model to obtain the scenario information, where the second prediction model is a model that is obtained by training a deep neural network by using the ACC sample data as an input and using the exercise scenario label as a target variable, and the second prediction model has the scenario identification function; and
input the PPG effective spectrum data and the obtained scenario information to a third prediction model to obtain the heart rate information, where the third prediction model is a model that is obtained by training the deep Attention network by using the PPG sample data, the ACC sample data, and the exercise scenario label as inputs and using the heart rate label as a target variable, and the third prediction model has the heart rate detection function.

The target prediction model includes the second prediction model and the third prediction model.

The foregoing solution provides another heart rate detection solution: An exercise scenario information identification function and a heart rate information identification function are separated. Scenario information identification is first performed by using a deep neural network model, and then an identification result and frequency domain sensor data are used as deep Attention inputs to output a heart rate value. The solution in this application can resolve a problem of various types of noise interference caused to a PPG signal in different scenarios, to effectively detect a heart rate in different noise scenarios.

In some possible implementations, the processing unit 820 is further configured to:
separately perform first preprocessing on the ACC signal and the PPG signal to obtain the ACC effective spectrum data and the PPG effective spectrum data.

The first preprocessing includes fast Fourier transform FFT and filtering processing.

According to the foregoing solution, FFT transform and filtering processing may be performed on a collected signal to obtain an effective spectrum, and a heart rate value is detected by using the effective spectrum. Because an interference signal is removed from the effective spectrum, precision of heart rate detection can be improved.

In some possible implementations, the obtaining unit 810 is specifically configured to collect the PPG signal by using a PPG sensor, and collect the ACC signal by using an acceleration sensor.

The PPG signal may be used to detect a heart rate, and the ACC signal may be used to identify an exercise scenario. According to the solution in this application, heart rate detection is performed by using the PPG signal and the ACC signal in combination, to resolve a problem of various types of noise interference caused to a PPG signal in different scenarios, thereby effectively detecting a heart rate in different noise scenarios.

In some possible implementations, the apparatus 800 may further include a transceiver unit 850. The transceiver unit 850 is configured to send the heart rate information and the scenario information to a terminal device, where the terminal device is an electronic device wirelessly connected to the wearable device, so that the heart rate information and the scenario information are displayed on a screen of the terminal device.

According to this solution, the heart rate information and the scenario information not only can be displayed by using the wearable device, but also can be displayed by using the terminal device connected to the wearable device, to help the user view a heart rate value, thereby improving use experience of a user.

The apparatus 800 according to this embodiment of this application may correspondingly perform the methods described in the embodiments of this application. In addition, the foregoing and other operations and/or functions of the units in the apparatus 800 are respectively configured to implement corresponding procedures of the methods. For brevity, details are not described herein again.

FIG. 11 is a schematic diagram of a structure of a wearable device 900 according to an embodiment of this application. The wearable device 900 includes a processor 910, a memory 920, a communication interface 930, and a bus 940.

The processor 910 may be connected to the memory 920. The memory 920 may be configured to store the program code and data. Therefore, the memory 920 may be an internal storage unit of the processor 910, may be an external storage unit independent of the processor 910, or may be a component including an internal storage unit of the processor 910 and an external storage unit independent of the processor 910.

Optionally, the wearable device 900 may further include the bus 940. The memory 920 and the communication interface 930 may be connected to the processor 910 by using the bus 940. The bus 940 may be a peripheral component interconnect (peripheral component interconnect, PCI) bus, an extended industry standard architecture (extended industry standard architecture, EISA) bus, or the like. The bus 940 may be classified into an address bus, a data bus, a control bus, and the like. For ease of representation, only one line is used for representation in FIG. 11, but this does not indicate that there is only one bus or one type of bus.

It should be understood that in this embodiment of this application, the processor 910 may be a central processing unit (central processing unit, CPU). The processor may alternatively be another general-purpose processor, a digital signal processor (digital signal processor, DSP), an application specific integrated circuit (application specific integrated circuit, ASIC), a field programmable gate array (field programmable gate Array, FPGA) or another programmable logic device, a discrete gate or a transistor logic device, a discrete hardware component, or the like. The general-purpose processor may be a microprocessor, or the processor may be any conventional processor or the like. Alternatively, the processor 910 may use one or more integrated circuits to execute a related program, to implement the technical solutions provided in the embodiments of this application.

The memory 920 may include a read-only memory and a random access memory, and provides instructions and data for the processor 910. A part of the processor 910 may further include a non-volatile random access memory. For example, the processor 910 may further store information about a device type.

When the wearable device 900 runs, the processor 910 executes computer-executable instructions in the memory 920 to perform the operation steps in the foregoing methods.

It should be understood that, the wearable device 900 according to this embodiment of this application may correspond to the apparatus 800 in the embodiments of this application, the processor 910 in the wearable device 900 may correspond to the processing unit 820 in the apparatus 800, the memory 920 in the wearable device 900 may correspond to the storage unit 840 in the apparatus 800, and the communication interface 930 in the wearable device 900 may correspond to the transceiver unit 850 in the apparatus 800. The foregoing and other operations and/or functions of the units in the apparatus 800 are respectively configured to implement corresponding procedures of the foregoing methods. For brevity, details are not described herein again.

Optionally, in some embodiments, an embodiment of this application further provides a computer-readable medium. The computer-readable medium stores program code. When the computer program code is run on a computer, the computer is enabled to perform the methods in the foregoing aspects.

Optionally, in some embodiments, an embodiment of this application further provides a computer program product. The computer program product includes computer program code. When the computer program code is run on a computer, the computer is enabled to perform the methods in the foregoing aspects.

In the embodiments of this application, the wearable device includes a hardware layer, an operating system layer running over the hardware layer, and an application layer running over the operating system layer. The hardware layer may include hardware such as a central processing unit (central processing unit, CPU), a memory management unit (memory management unit, MMU), and a memory (also referred to as a main memory). An operating system at the operating system layer may be any one or more computer operating systems that implement service processing by using a process (process), for example, a Linux operating system, a Unix operating system, an Android operating system, an iOS operating system, or a windows operating system. The application layer may include applications such as a browser, an address book, text processing software, and instant messaging software.

A specific structure of an entity for performing the method provided in the embodiments of this application is not specially limited in the embodiments of this application, provided that a program that records code of the method provided in the embodiments of this application can be run to perform communication based on the method provided in the embodiments of this application. For example, the method provided in the embodiments of this application may be performed by a wearable device, or a functional module that is in a wearable device and that can invoke a program and execute the program.

Aspects or features of this application may be implemented as a method, an apparatus, or a product that uses standard programming and/or engineering technologies. The term "product" used in this specification may cover a computer program that can be accessed from any computer-readable component, carrier, or medium. For example, the computer-readable medium may include but is not limited to a magnetic storage component (for example, a hard disk, a floppy disk, or a magnetic tape), an optical disc (for example, a compact disc (compact disc, CD) or a digital versatile disc (digital versatile disc, DVD)), a smart card, and a flash memory device (for example, an erasable programmable read-only memory (erasable programmable read-only memory, EPROM), a card, a stick, or a key driver).

Various storage media described in this specification may indicate one or more devices and/or other machine-readable media that are configured to store information. The term "machine-readable media" may include but is not limited to a radio channel and various other media that can store, include, and/or carry instructions and/or data.

It should be understood that, the processor mentioned in the embodiments of this application may be a central processing unit (central processing unit, CPU), or may be another general-purpose processor, a digital signal processor (digital signal processor, DSP), an application specific integrated circuit (application specific integrated circuit, ASIC), a field programmable gate array (field programmable gate array, FPGA) or another programmable logic device, a discrete gate or a transistor logic device, a discrete hardware component, or the like. The general-purpose processor may be a microprocessor, or the processor may be any conventional processor or the like.

It should be further understood that, the memory mentioned in the embodiments of this application may be a volatile memory or a non-volatile memory, or may include both a volatile memory and a non-volatile memory. The non-volatile memory may be a read-only memory (read-only memory, ROM), a programmable read-only memory (programmable ROM, PROM), an erasable programmable read-only memory (erasable PROM, EPROM), an electrically erasable programmable read-only memory (electrically EPROM, EEPROM), or a flash memory. The volatile memory may be a random access memory (random access memory, RAM). For example, the RAM may be used as an external cache. For example but not for limitation, the RAM may include the following a plurality of forms: a static random access memory (static RAM, SRAM), a dynamic random access memory (dynamic RAM, DRAM), a synchronous dynamic random access memory (synchronous DRAM, SDRAM), a double data rate synchronous dynamic random access memory (double data rate SDRAM, DDR SDRAM), an enhanced synchronous dynamic random access memory (enhanced SDRAM, ESDRAM), a synchlink dynamic random access memory (synchlink DRAM, SLDRAM), and a direct rambus random access memory (direct rambus RAM, DR RAM).

It should be noted that when the processor is a general-purpose processor, a DSP, an ASIC, an FPGA or another programmable logic device, a discrete gate or a transistor logic device, or a discrete hardware component, the memory (storage module) may be integrated into the processor.

It should be further noted that the memory described in this specification is intended to include but not limited to these memories and any other proper types of memories.

A person of ordinary skill in the art may be aware that, example units and steps described with reference to the embodiments disclosed in this specification can be implemented by electronic hardware or a combination of computer software and electronic hardware. Whether these functions are performed by hardware or software depends on specific applications and design constraints of the technical solutions. A skilled person may use different methods to implement the described functions for each specific application, but it should not be considered that the implementation goes beyond the protection scope of this application.

A person skilled in the art may clearly understand that, for the purpose of convenient and brief description, for a specific working process of the system, apparatus, and unit described above, refer to a corresponding process in the foregoing method embodiments. Details are not described herein again.

In the several embodiments provided in this application, it should be understood that the disclosed system, apparatus, and method may be implemented in other manners. For example, the apparatus embodiments described above are merely examples. For example, the unit division is merely logical function division, and there may be another division manner in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in an electronic form, a mechanical form, or another form.

The units described as separate components may or may not be physically separated, and components displayed as units may or may not be physical units, that is, may be located in one place, or may be distributed on a plurality of network units. Some or all of the units may be selected based on an actual requirement, to achieve the objectives of the solutions in the embodiments.

In addition, functional units in embodiments of this application may be integrated into one unit, or each of the units may exist alone physically, or two or more units may be integrated into one unit.

When the function is implemented in a form of a software functional unit and sold or used as an independent product, the function may be stored in a computer-readable storage medium. Based on such an understanding, the technical solutions of this application essentially, or the part contributing to the conventional technology, or some of the technical solutions may be implemented in a form of a computer software product. The computer software product is stored in a storage medium. The computer software product includes several instructions for instructing a computer device (which may be a personal computer, a server, a network device, or the like) to perform all or some of the steps of the methods described in the embodiments of this application. The storage medium may include but is not limited to any medium that can store program code, for example, a USB flash drive, a removable hard disk, a ROM, a RAM, a magnetic disk, or an optical disc.

Unless otherwise specified, all technical and scientific terms used in this specification have meanings the same as those commonly understood by a person skilled in the art of this application. In this specification, the terms used in the specification of this application are merely intended to describe objectives of specific embodiments, but are not intended to limit this application.

The foregoing descriptions are merely specific implementations of this application, and are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A deep learning-based heart rate detection method, comprising:
when a user wears a wearable device, obtaining a photoplethysmography PPG signal and an acceleration ACC signal in response to a heart rate detection command;
obtaining heart rate information and scenario information based on ACC effective spectrum data corresponding to the ACC signal, PPG effective spectrum data corresponding to the PPG signal, and a target prediction model; and
displaying the heart rate information and the scenario information on a screen of the wearable device, wherein
the target prediction model is a model that is obtained by training a deep attention Attention network by using ACC sample data and PPG sample data as inputs and using a heart rate label and an exercise scenario label as target variables, and the target prediction model has a scenario identification function and a heart rate prediction function.

2. The method according to claim 1, wherein the method further comprises:
when the heart rate information and the scenario information are obtained, recording the heart rate information and the scenario information in a label buffer; and
recording, in a data buffer, spectrum peak-point data corresponding to the heart rate information and the scenario information, wherein
the spectrum peak-point data corresponding to the heart rate information and the scenario information comprises a peak point location and an amplitude that are of a PPG spectrum and a peak point location and an amplitude that are of an ACC spectrum.

3. The method according to claim 2, wherein the obtaining heart rate information and scenario information based on ACC effective spectrum data corresponding to the ACC signal, PPG effective spectrum data corresponding to the PPG signal, and a target prediction model comprises:
obtaining first N pieces of peak-point spectrum data based on the PPG effective spectrum data and the ACC effective spectrum data; and
if no peak-point spectrum data in the first N pieces of peak-point spectrum data is prestored in the data buffer, obtaining the heart rate information and the scenario information based on the ACC effective spectrum data corresponding to the ACC signal, the PPG effective spectrum data corresponding to the PPG signal, and the target prediction model; or
if any peak-point spectrum data in the first N pieces of peak-point spectrum data is prestored in the data buffer, reading, from the label buffer, heart rate information and scenario information that correspond to the any peak-point spectrum data.

4. The method according to any one of claims 1 to 3, wherein the obtaining heart rate information and scenario information based on ACC effective spectrum data corresponding to the ACC signal, PPG effective spectrum data corresponding to the PPG signal, and a target prediction model comprises:
inputting the ACC effective spectrum data and the PPG effective spectrum data to a first prediction model to obtain the heart rate information and the scenario information, wherein
the target prediction model is the first prediction model, the first prediction model is a model that is obtained by training the deep attention Attention network by using the ACC sample data and the PPG sample data as inputs and using the heart rate label and the exercise scenario label as target variables, and the first prediction model has the scenario identification function and the heart rate prediction function.

5. The method according to any one of claims 1 to 3, wherein the obtaining heart rate information and scenario information based on ACC effective spectrum data corresponding to the ACC signal, PPG effective spectrum data corresponding to the PPG signal, and a target prediction model comprises:
inputting the ACC effective spectrum data to a second prediction model to obtain the scenario information, wherein the second prediction model is a model that is obtained by training a deep neural network by using the ACC sample data as an input and using the exercise scenario label as a target variable, and the second prediction model has the scenario identification function; and
inputting the PPG effective spectrum data and the obtained scenario information to a third prediction model to obtain the heart rate information, wherein the third prediction model is a model that is obtained by training the deep Attention network by using the PPG sample data, the ACC sample data, and the exercise scenario label as inputs and using the heart rate label as a target variable, and the third prediction model has the heart rate detection function, wherein
the target prediction model comprises the second prediction model and the third prediction model.

6. The method according to any one of claims 1 to 5, wherein after the obtaining a PPG signal and an ACC signal, the method further comprises:
separately performing first preprocessing on the ACC signal and the PPG signal to obtain the ACC effective spectrum data and the PPG effective spectrum data, wherein
the first preprocessing comprises fast Fourier transform FFT and filtering processing.

7. The method according to any one of claims 1 to 6, wherein the obtaining a PPG signal and an ACC signal comprises:
collecting the PPG signal by using a PPG sensor, and collecting the ACC signal by using an acceleration sensor.

8. The method according to any one of claims 1 to 6, wherein the method further comprises:
sending the heart rate information and the scenario information to a terminal device, wherein the terminal device is an electronic device wirelessly connected to the wearable device; and
displaying the heart rate information and the scenario information on a screen of the terminal device.

9. A method for training a model used for heart rate detection, comprising:
obtaining a multi-scenario sample set, wherein the multi-scenario sample set is a data sample set obtained through detection based on a plurality of exercise scenarios;
extracting ACC sample data, PPG sample data, and a heart rate label from the multi-scenario sample set;
training a deep attention Attention network by using the ACC sample data and the PPG sample data as inputs and using the heart rate label and an exercise scenario label as target variables; and
obtaining a first prediction model, wherein the first prediction model has a scenario identification function and a heart rate prediction function.

10. The method according to claim 9, wherein the training a deep Attention network by using the ACC sample data and the PPG sample data as inputs and using the heart rate label and an exercise scenario label as target variables comprises:
adjusting a parameter of a model through cross-validation, so that the model learns to predict heart rate information in different exercise scenarios.

11. The method according to claim 9 or 10, wherein the obtaining a multi-scenario sample set comprises:
connecting a wearable device and a heart rate band device to a data collection module;
when a user wearing the wearable device and the heart rate band device does a first exercise, obtaining, by the data collection module, first heart rate detection data of the wearable device and second heart rate detection data of the heart rate band device; and
when the user does a second exercise, obtaining, by the data collection module, third heart rate detection data of the wearable device and fourth heart rate detection data of the heart rate band device, wherein
the first exercise and the second exercise are exercises indicated by the exercise scenario label, and the multi-scenario sample set comprises the first heart rate detection data, the second heart rate detection data, the third heart rate detection data, and the fourth heart rate detection data.

12. The method according to claim 11, wherein the extracting ACC sample data and PPG sample data from the multi-scenario sample set comprises:
extracting the ACC sample data and the PPG sample data from detection data of the wearable device, wherein
the detection data of the wearable device comprises the first heart rate detection data and the third heart rate detection data.

13. The method according to claim 11, wherein the extracting a heart rate label from the multi-scenario sample set comprises:
extracting the heart rate label from detection data of the heart rate band device, wherein
the detection data of the heart rate band device comprises the second heart rate detection data and the fourth heart rate detection data.

14. The method according to any one of claims 9 to 13, wherein after the extracting ACC sample data, PPG sample data, and a heart rate label from the multi-scenario sample set, the method further comprises:
performing fast Fourier transform FFT and filtering processing on the ACC sample data and the PPG sample data to obtain filtered ACC sample data and PPG sample data; and
the training a deep Attention network by using the ACC sample data and the PPG sample data as inputs and using the heart rate label and an exercise scenario label as target variables comprises:
training the deep Attention network by using the filtered ACC sample data and PPG sample data as inputs and using the heart rate label and the exercise scenario label as target variables.

15. The method according to claim 14, wherein the filtering processing is used to filter out noise data outside [0.7 Hz, 4 Hz] in a spectrum.

16. The method according to any one of claims 9 to 15, wherein after the obtaining a first prediction model, the method further comprises:
quantizing the first prediction model by using a preset quantization parameter, to obtain a quantized first prediction model.

17. A method for training a model used for heart rate detection, comprising:
obtaining a multi-scenario sample set, wherein the multi-scenario sample set is a data sample set obtained through detection based on a plurality of exercise scenarios;
extracting ACC sample data, PPG sample data, and a heart rate label from the multi-scenario sample set;
training a deep attention Attention network by using the ACC sample data, the PPG sample data, and an exercise scenario label as inputs and using the heart rate label as a target variable; and
obtaining a third prediction model, wherein the third prediction model has a heart rate prediction function.

18. The method according to claim 17, wherein the method further comprises:
training a deep neural network by using the ACC sample data and the PPG sample data as inputs and using the exercise scenario label as a target variable, to obtain a second prediction model, wherein the second prediction model has a scenario identification function.

19. The method according to claim 17 or 18, wherein the training a deep Attention network by using the ACC sample data, the PPG sample data, and an exercise scenario label as inputs and using the heart rate label as a target variable comprises:
adjusting a parameter of an Attention network model through cross-validation, so that the Attention network model learns to predict heart rate information in different exercise scenarios.

20. The method according to any one of claims 17 to 19, wherein the obtaining a multi-scenario sample set comprises:
connecting a wearable device and a heart rate band device to a data collection module;
when a user wearing the wearable device and the heart rate band device does a first exercise, obtaining, by the data collection module, first heart rate detection data of the wearable device and second heart rate detection data of the heart rate band device; and
when the user does a second exercise, obtaining, by the data collection module, third heart rate detection data of the wearable device and fourth heart rate detection data of the heart rate band device, wherein
the first exercise and the second exercise are exercises indicated by the exercise scenario label, and the multi-scenario sample set comprise the first heart rate detection data, the second heart rate detection data, the third heart rate detection data, and the fourth heart rate detection data.

21. The method according to claim 20, wherein the extracting ACC sample data and PPG sample data from the multi-scenario sample set comprises:
extracting the ACC sample data and the PPG sample data from detection data of the wearable device, wherein
the detection data of the wearable device comprises the first heart rate detection data and the third heart rate detection data.

22. The method according to claim 20, wherein the extracting a heart rate label from the multi-scenario sample set comprises:
extracting the heart rate label from detection data of the heart rate band device, wherein
the detection data of the heart rate band device comprises the second heart rate detection data and the fourth heart rate detection data.

23. The method according to any one of claims 17 to 22, wherein after the extracting ACC sample data, PPG sample data, and a heart rate label from the multi-scenario sample set, the method further comprises:
performing fast Fourier transform FFT and filtering processing on the ACC sample data and the PPG sample data to obtain filtered ACC sample data and PPG sample data; and
the training a deep Attention network by using the ACC sample data, the PPG sample data, and an exercise scenario label as inputs and using the heart rate label as a target variable comprises:
training the deep Attention network by using the filtered ACC sample data and PPG sample data and the exercise scenario label as inputs and using the heart rate label as a target variable.

24. The method according to claim 23, wherein the filtering processing is used to filter out noise data outside [0.7 Hz, 4 Hz] in spectrum data corresponding to the ACC sample data and the PPG sample data.

25. The method according to any one of claims 17 to 24, wherein after the obtaining a third prediction model, the method further comprises:
quantizing the third prediction model by using a preset quantization parameter, to obtain a quantized third prediction model.

26. A wearable device, comprising a processor, wherein the processor is coupled to a memory, and the processor is configured to execute a computer program or instructions stored in the memory, so that the wearable device implements the method according to any one of claims 1 to 25.

27. A chip, wherein the chip is coupled to a memory, and the chip is configured to read and execute a computer program stored in the memory, to implement the method according to any one of claims 1 to 25.

28. A computer-readable storage medium, wherein the computer-readable storage medium stores a computer program, and when the computer program is run on a wearable device, the wearable device is enabled to perform the method according to any one of claims 1 to 25.
